# EUROPEAN PATENT APPLICATION

(11) **EP 4 202 059 A1**
(43) Date of publication of application: **28.06.2023**
(21) Application number: 21217005.4
(22) Date of filing: 22.12.2021
(51) Int. Cl.: C12Q 1/6883

(54) **METHOD FOR DETERMINING A CIRCADIAN RHYTHM TYPE OF A HUMAN SUBJECT**

(71) Applicant: Charité - Universitätsmedizin Berlin, 10117 Berlin (DE)
(72) Inventor: Kramer, Achim, Berlin (DE)
(74) Representative: Hertin und Partner Rechts- und Patentanwälte PartG mbB

(57) **Abstract**

The invention relates to a method for determining a circadian rhythm of a subject, comprising providing a sample from a subject, such as a hair root, oral mucosa, or a saliva sample, quantifying RNA expression levels of circadian phase genes and amplitude prediction genes in said sample, and applying the expression levels in a mathematical model. The invention further relates to diagnostic and/or medical uses of the invention, and corresponding therapeutic methods, comprising provision of optimal and/or recommended physical activity times based on the circadian rhythm, such as times recommended for sleeping, working, meal intake, sport, intellectual tasks, light exposure, medical intervention, and/or medical diagnostics. In further aspects, the invention relates to a computer-readable storage medium adapted to configure a processor to perform a method comprising executing a biomathematical model algorithm based on the method of the invention.

## Description

The invention relates to the field of circadian rhythms, methods for determining circadian rhythms and related materials, devices, diagnostic methods and devices, and applied mathematical models.

The present invention therefore relates to a method for determining a circadian rhythm of a subject, comprising providing a sample from a subject, such as a hair root, oral mucosa, or a saliva sample, quantifying RNA expression levels of circadian phase genes and amplitude prediction genes in said sample, and applying the expression levels in a mathematical model. In some embodiments the invention relates to a method for determining a circadian rhythm of a human subject, comprising providing a single sample comprising RNA from said subject, and quantifying an RNA expression level of multiple genes in said sample, said multiple genes comprising at least one gene selected from at least two of the following circadian phase gene groups: an early-day gene group, a day gene group, an evening gene group, and a night gene group, and at least one gene selected from a group of amplitude prediction genes, and determining the circadian rhythm based on the expression levels quantified from said single sample.

The invention further relates to diagnostic and/or medical uses of the invention, and corresponding therapeutic methods, comprising provision of optimal and/or recommended physical activity times based on the circadian rhythm, such as times recommended for sleeping, working, meal intake, sport, intellectual tasks, light exposure, medical intervention, and/or medical diagnostics.

In further aspects, the invention relates to a computer-readable storage medium adapted to configure a processor to perform a method comprising executing a biomathematical model algorithm with input data including quantitative RNA expression values from said subject and a training data set, such as gene label, a weight factor, and an amplitude score and/or time parameters from other subjects, to produce a circadian rhythm of said subject as an output.

### BACKGROUND OF THE INVENTION

As a molecular time-keeping mechanism, the internal circadian clock generates a biological rhythm and regulates various physiological processes such as blood pressure, hormone secretion, sleep-wake cycles, and body temperature in mammals, and in particular, in humans. Circadian clocks are highly conserved throughout evolution. In mammals, the main pacemaker is located in the suprachiasmatic nucleus (SCN). The SCN consists of multiple, single-cell circadian oscillators that operate cell autonomously. They are synchronized to fire rhythmically and, when intact, produce a coordinated circadian rhythmic output.

In our modern times and as a result of diverse and sometimes unnatural lifestyles or living circumstances, such as externally regulated working hours and cross-time zone flights, many people live out of sync with their individual circadian rhythms, with serious consequences for the quality of life, health, and daily performance in work and learning abilities. Thus, mismatched or disrupted circadian clocks are common in modern society, and can be detrimental to health and quality of life. It has been shown that living against one's individual body clock puts people at high risk for numerous chronic diseases, including chronic sleep disorders, cancer, cardiovascular and metabolic diseases, and psychiatric disorders, such as depression. In addition, daytime adjusted therapeutic intervention (chronotherapy) has been shown to be superior to standard therapy, for example, in cancer chemotherapy or rheumatoid arthritis.

The body clock of each person is a permanently running a cell-based molecular program being largely determined by his genetics. That is why there are different types in our society - so-called "morning larks" (early chronotypes), normal chronotypes or so-called "night-owls" (late chronotypes). To live according to one's own body clock seems to be a simple and plausible principle for keeping a person healthy. However, for example, 70% of the adult population in Germany fails to do so. Social and economic reasons are primary factors. This condition is often called "social jetlag", because the socially oriented clock (e.g. indicated by a wristwatch) does not correspond to the individual body clock. An early riser is still considered hardworking and successful in the social environment. As a result, people constantly push their bodies to do things at times when they are not naturally ready to do them. However, most of us have become so accustomed to living against our individual biological clocks that we don't necessarily realize the damage we are doing to our bodies.

There is still a lack of simple and convenient diagnostic tools to characterize the human chronobiological state, i.e., internal time and strength of rhythms. Such tools are urgently needed. Similar analytical tools would enable various advantages, capable of advancing chronomedicine (also called circadian medicine) as a field, e.g., (i) to personalize chronotherapy according to the individual's internal time (chronotype); (ii) to distribute work schedules to day or night shifts according to the employee's chronotype, thereby minimizing disruptions to the internal clock; (iii) to develop treatment strategies (e.g., light or melatonin therapy) for patients with internal rhythm disorders (e.g. ICU patients, patients with neurodegenerative diseases, elderly); (iv) to learn more about the interaction of the circadian system with common diseases; (v) to stratify cohorts eg. ICU patients, neurodegenerative disease patients, elderly); (iv) to learn more about the interaction of the circadian system with common diseases; (v) promoting health and preventing disease (vi) as well as to stratify cohorts in clinical trials by internal time (as the pharmacokinetics and pharmacodynamics of many drugs have been shown to be time-of-day dependent). Furthermore, such analytical tools would give people easy access to a methodology to check their own chronobiological state and adjust their lifestyle, and thus avoid or intervene in body-clock disrupting activity.

To enable a harmonious lifestyle, in sync with one's internal body clock, analytical tools need to be available for end-users with little complication or cost. However, until now it has been almost impossible to accurately determine the individual body clock (chronotype), in a reliable and accessible manner. Currently, the only way of chronotyping an individual in the prior art (i.e., assessing his or her individual internal time) is done either (i) by questionnaires, (ii) by determining physiological or behavioral parameters using many repeated measurements (time series) or (iii) by determining parameters in samples of a human subject that are not easily accessible. Additionally, such prior art methods typically provide results that only imprecisely reflect the present chronobiological state.

Questionnaires, such as the Munich Chronotype Questionnaire or the Home & Ostberg morningness-eveningness questionnaire, are by nature lacking in objectivity, as they depend on an individual's own statements. Moreover, they do not capture a chronobiological state, but ask for a general preference or description of sleep habits.

In contrast, the determination of physiological or behavioral parameters is a more objective alternative to the measurement of rhythmic processes and the determination of a circadian rhythm. Examples include actigraphy, which uses portable devices to determine a person's activity profile over several days. In addition, core body temperature rhythms are determined with appropriate temperature probes in a controlled clinical setting. Currently, the most commonly used method to objectively assess an individual's internal phase is to determine the so-called dim-light melatonin onset (DLMO) (Pandi-Perumal SR et al, Prag Neuropsychopharmacol Biol Psychiatry). For this purpose, subjects must stay in dim light for 6 h in the evening and give a saliva or blood sample every 30 min, which is then analyzed for melatonin levels. This is a lengthy and complicated procedure that cannot be used in routine medical practice (usually in sleep laboratories). Although this method is considered the gold standard for determining chronotype, it is rarely used in clinical practice because of the stressful, uncomfortable, time-consuming, and also very expensive procedure.

None of the solutions available in the art are easy to perform and provide valid and reproducible results without requiring a specialized environment. The objective of the present invention addresses an unmet and high medical need in addressing individual lifestyle aspects and health care of circadian rhythm related disorders.

### SUMMARY OF THE INVENTION

In light of the prior art, the technical problem underlying the invention was the provision of alternative or improved means for determining an amplitude and a phase of the circadian rhythm of a human subject. RNA expression levels of genes associated to circadian phase, such as an early-day gene, a day gene, an evening gene, and a night gene, and amplitude prediction genes, are determined in samples from a subject, such as a hair root, and are applied to an amplitude of the circadian rhythm of the subject.

Another objective was to provide a method to process a sample from a subject for determining individual expression levels of circadian phase and amplitude prediction genes for computing a subject's phase of circadian rhythm, and amplitude of the circadian rhythm, by using a biomathematical model trained with a reference data set.

The present invention therefore provides means and methods for a simple, accurate and fast calculation of an amplitude and a phase of the circadian rhythm of a human subject using a biomathematical model. In some embodiments, the invention enables circadian rhythm determination by method of the invention and provides an assessment and/or grading of the lifestyle of a human subject. One objective of the invention was to provide a computational method to determine a circadian rhythm used for grading and recommendation of the human subject's lifestyle and/or for preventing, prognosis, ascertaining, and/or treating disorder associated with circadian rhythm disruption or misalignment. Another objective of the invention was to provide a computational method to determine a circadian rhythm used for treating a circadian rhythm related disorder.

Despite the high medical need, the prior art currently does not provide means to determine the phase of circadian rhythm and amplitude of the circadian rhythm of a subject, particularly by using individual hair root samples, and thus to assess optimal lifestyle and/or recommended physical activity times.

The technical problem can also be seen in the provision of means to determine an amplitude and a phase of the circadian rhythm of a subject by combining biomathematical modeling with RNA expression levels of circadian phase genes determined in the sample. The technical problem can also be seen in providing means to execute a mathematical modeling algorithm by a computer program.

These problems are solved by the features of the independent claims. Preferred embodiments of the present invention are provided by the dependent claims.

The invention therefore relates to a method for determining a circadian rhythm of a human subject, comprising
a. Providing a single sample comprising RNA from said subject, and
b. Quantifying an RNA expression level of multiple genes in said sample, said multiple genes comprising at least one gene selected from at least two of the following circadian phase gene groups: an early-day gene group, a day gene group, an evening gene group, and a night gene group, and at least one gene selected from a group of amplitude prediction genes, and
c. Determining the circadian rhythm based on the expression levels quantified in step b from said single sample.

In one embodiment, a single sample is provided by the human subject taken at any time of day or taken at a specific time of a day.

In some embodiments, more than one sample is provided by the human subject taken at any time of day with a regular time interval between each sampling.

From each sample, RNA is extracted, as described in non-limiting terms in the Examples. The RNA expression level is also determined, as described in non-limiting terms in the Examples. In embodiments, RNA levels are determined for each gene selected from at least two of the circadian phase gene groups and at least one gene selected from the amplitude prediction genes group.

The invention provides means and methods for determining a circadian phase and an amplitude of the circadian rhythm in a human subject based on the expression levels. The circadian phase and the amplitude are characteristic for a circadian rhythm of the subject. In some embodiments, the amplitude corresponds to the strength of a person's circadian rhythm.

In one embodiment, the circadian rhythm is determined based on the RNA expression levels quantified for at least two genes selected from the early-day gene group, the day gene group, and at least one gene selected from the amplitude prediction gene group.

In one embodiment, the circadian rhythm is determined based on the RNA expression levels quantified for at least two genes selected from the early-day gene group, evening gene group, and at least one gene selected from the amplitude prediction gene group.

In one embodiment, the circadian rhythm is determined based on the RNA expression levels quantified for at least two genes selected from the early-day gene group, the night gene group, and at least one gene selected from the amplitude prediction gene group.

In one embodiment, the circadian rhythm is determined based on the RNA expression levels quantified for at least two genes selected from the day gene group, the evening gene group, and at least one gene selected from the amplitude prediction gene group.

In one embodiment, the circadian rhythm is determined based on the RNA expression levels quantified for at least two genes selected from the day gene group, the night gene group, and at least one gene selected from the amplitude prediction gene group.

In one embodiment, the circadian rhythm is determined based on the RNA expression levels quantified for at least two genes selected from the evening gene group, the night gene group, and at least one gene selected from the amplitude prediction gene group.

The present invention also provides means and methods for predicting circadian rhythm or circadian rhythm phase shift disturbances caused by an internal clock disturbance, such as circadian rhythm sleep disturbance, and more particularly, to provide a method for accurate and simple diagnosis of circadian rhythm disturbance.

In some embodiments, the circadian rhythm is determined based on the RNA expression levels quantified for at least 3, 4, 5, 6, 7, 8, 9, 10, more than 10, more than 20, more than 30, more than 40, more than 50, more than 60, more than 70, more than 80 or more than 90 genes. In one embodiment, the circadian rhythm is determined based on the RNA expression levels quantified for 80 to 120 genes, such as 96 genes, as demonstrated in the non-limiting examples.

The genes in the groups provided herein are particularly advantageous for determining circadian rhythm with high accuracy. The accuracy can be 3h, 2h, 1h, 45min, 30min, 15min or less. In some embodiments, the accuracy refers to the precision, or difference, between the measured circadian rhythm and the actual circadian rhythm of the subject.

In some embodiments, the sample is a hair root, oral mucosa, or saliva sample, preferably a hair root sample.

In one embodiment, the sample is a blood sample, such as whole blood, plasma, or serum sample (plasma from which clotting factors have been removed). For example, peripheral, arterial or venous plasma or serum may be used. In another embodiment, the sample is urine, sweat, or other body fluid in which proteins are sometimes removed from the bloodstream.

In one embodiment, the sample is a skin sample.

In one embodiment, the sample is a hair root sample. Hair root samples are particularly suited for sampling because they are painless, easy to obtain, durable, can be easily shipped by the subject, and are simple to process for RNA extraction. Sampling, RNA extraction and RNA expression analysis of hair root samples are described in non-limiting terms in the Examples.

In preferred embodiments, the RNA expression level of genes selected from the circadian phase gene group, and from the amplitude prediction gene group, is determined in one or more hair roots, wherein a sample comprising one or more hair roots obtained at essentially the same time is considered a single sample of the subject. In some embodiments, said RNA expression level are determined in each of two or more samples from the same patient. In some embodiments, two or more samples are obtained at a time-of-day or night, wherein each sample is taken 2 - 10 hours apart. In some embodiments, two or more samples are obtained at a time-of-day that is 3h, 4h, 6h, 7h, 8h or 9h apart.

In some embodiments, the method comprises determining an amplitude and a phase of the circadian rhythm of the subject.

In preferred embodiments, the single hair root sample is used for determining the amplitude and the circadian phase of the circadian rhythm of the subject.

Therefore, the strength of a person's circadian rhythm can also be determined by the expression level of amplitude prediction genes as a measurement of the amplitude of a circadian oscillatory variable. For example, such circadian oscillatory variables include the expression level of a circadian oscillatory gene or the expression level of a set of circadian oscillatory genes, hormone levels (e.g., cortisol), or the behavioral activity level determined by actigraphy. In actigraphy, a person's activity profile is determined over several days using wearable devices. A large amplitude of a circadian oscillation variable is indicative of a strong individual circadian rhythm and a small amplitude is indicative of a weak circadian rhythm.

In one embodiment, the present invention uses quantitative analysis of RNA expression levels of genes defined as circadian phase genes. In one embodiment, the peak of RNA expression of the circadian phase genes corresponds to specific times over a 24-hour period. In one embodiment, the oscillation of circadian phase gene transcripts has its maximum at a time or time period of the day. The circadian phase genes are therefore grouped according to their peak expression phases. For example, circadian phase genes are grouped into early day gene, day gene, evening gene, and night gene groups. In one embodiment, the circadian phase gene transcripts can be detected at other times of the day. In one embodiment, the circadian phase gene transcripts are undetectable at other times of the day.

In some embodiments, the method comprises normalizing the determined expression level of each circadian phase gene to an expression level of at least one housekeeping gene or to a mean expression level of all determined gene expression levels. Other forms or means for normalization are known to a skilled person and are not excluded from the invention.

In some embodiments, the RNA is quantified by quantitative PCR, microarray, sequencing or NanoString technology. In one embodiment, the normalization of the amount of cellular RNA in the sample is performed via one or more housekeeping genes. The housekeeping gene is also quantitatively determined and used for the normalization of the amount of cellular RNA in the sample of said subject. In one embodiment, a housekeeping gene is preferably selected from the group consisting of GAPDH, PGK1, CLTC, PSMB2, ACTB (beta-Actin), 18s rRNA, PPIA, UBC, EIF4E2 and HPRT1, or any other known housekeeping gene. In preferred embodiments, the normalization of the amount of cellular RNA in the sample is performed through determining the mean expression level of all determined gene expression levels, wherein said normalization is preferably performed when NanoString technolology is used for RNA quantification.

In some embodiments, the method comprises comparing the quantified (optionally normalized) RNA expression level of each gene to a predetermined gene expression level (such as maximum or minimum) for each gene and a corresponding phase in said human subject.

In one embodiment, the circadian phase and the amplitude determined in a sample at a specific time point of a diseased subject (patient, affected) can be compared to the circadian phase and the amplitude of a non-diseased subject (control, normal).

A further advantage of the invention is that the mathematical modelling can be performed for a plurality of sample types and time points of sampling, wherein the mathematical model of the invention can be adapted to the sample type. This increases the accuracy of the circadian phase and amplitude, in particular the accuracy of the prediction for circadian rhythm disturbance or treatment selection.

In one embodiment, the RNA expression levels, the sampling time point and/or parameter of the subject are introduced into the mathematical model.

In one embodiment, the quantitatively determined RNA expression of circadian phase genes and amplitude prediction genes of a diseased subject (patient, affected) can be compared to the quantitatively determined RNA expression of circadian phase genes and amplitude prediction genes of a non-diseased subject (control, normal).

In some embodiments, the method comprises determining an amplitude of the circadian rhythm of the subject, wherein the expression level of the one or more genes selected from the group of amplitude prediction genes correlates with an amplitude of the circadian rhythm of the subject.

As used herein, the amplitude of a circadian phase describes the difference in the level between peak and trough values and refers to the strength of a circadian oscillator. As known in the art, for determining the amplitude, the RNA expression level from the same gene needs to be determined in each of two or more samples from the same subject. As described herein, the invention provides means and methods for determining the amplitude from a single sample of a subject. The invention provides a combination of genes which has not been known in the art to be useful for determining the amplitude. It could not have been expected by a skilled person that one or more genes of the amplitude prediction gene group would correlate to the amplitude when using a single sample.

Amplitude prediction genes are, in embodiments, typically not rhythmic, but vary in expression strength. Some amplitude prediction genes may have a low expression level when the amplitude is weak. Some amplitude prediction genes may have a low expression level when the amplitude is strong. Some amplitude prediction genes may have a high expression level when the amplitude is weak. Some amplitude prediction genes may have a high expression level when the amplitude is strong. The determination of the amplitude predictions genes is described in non-limiting terms in the Examples.

In one embodiment, the circadian phase and the amplitude can be determined at a single time point. In one embodiment, the circadian phase and the amplitude can be determined using more than one time point.

In one embodiment, the amplitude value corresponds to a strength of circadian rhythm.

In some embodiments, the amplitude provides an assessment and/or grading of the lifestyle of the human subject in relation to the determined circadian rhythm.

The determination of the circadian rhythm and the degree of matching of the individual life style to the circadian rhythm and thus the rhythm strength are important to identify subjects with disturbed or weak internal rhythms (e.g. patients with sleep disorders, depression, dementia, in intensive care units, patients with neurodegenerative diseases, elderly people). Such patients particularly benefit from chronotherapy, i.e., treatment that supports the circadian rhythm (e.g., light or melatonin therapy, time-restricted eating, scheduled exercise).

In one embodiment, grading the lifestyle of a subject in relation to the determined circadian rhythm comprises a matching of the circadian rhythm type and corresponding circadian phases of the subject with their physical activity times.

In one embodiment, the determined circadian rhythm type matches the lifestyle of the subject. In one embodiment, the grading of the subject's lifestyle relates to a disturbed circadian rhythm. In one embodiment, the grading of the subject's lifestyle relates to circadian rhythm related disorders.

Determination of rhythm strength is also important for diagnostic purposes, as disturbed and/or weak internal rhythms are associated with various pathologies. As provided in the Examples, means and methods provided herein can be used for prevention, diagnosis and/or therapy of circadian rhythm related disorders.

For the decision on therapy, and selection of a suitable form of therapy, it is particularly advantageous to know the strength of the circadian rhythm and thus the degree of deviation from the circadian rhythm. The more precise the indication of the degree of deviation and the classification to a circadian rhythm type, the easier is the choice of therapy and the stronger is the likelihood of therapy success. This advantage is also important for the prevention of diseases related to circadian rhythm disorders. Therefore, the high accuracy provided by the invention is of decisive and medical advantage. Easy, painless, and non-invasive sampling, as described herein, is of particular advantage as this allows easy access to circadian rhythm determination, provided herein, to all subjects, for example, including pain sensitive people, hemophiliacs, etc. People who have no hair can also provide another sample type for circadian rhythm determination, such as a saliva sample.

Surprisingly, with only one sample, the amplitude can be determined correctly using the method as described herein. Typically, one needs two or more samples taken at different times of the day to correctly determine the amplitude. More samples have the disadvantage of extra effort and cost for the subject, for processing and analysis of the samples, and thus a prolonged turnaround time until the subject receives the analysis result. Thus, using the means and methods provided herein, samples can be analyzed in a shorter period of time to determine the circadian rhythm type and grading of the lifestyle of the human subject in relation to the determined circadian rhythm.

In some embodiments, the circadian rhythm of the human subject is determined as one or more circadian rhythm types with a defined circadian phase of optimal and/or recommended physical activity of the subject.

In some embodiments, a circadian rhythm type comprise an early morning type, a late morning type, an early afternoon type, a late afternoon type, an early evening type, a late evening type, an early night type, and a late night type.

In one embodiment, the circadian rhythm type early morning has an optimal and/or recommended time to conduct a physical activity between 6 am and 9 am.

In one embodiment, the circadian rhythm type late morning has an optimal and/or recommended time to conduct a physical activity between 9 am and 12 am.

In one embodiment, the circadian rhythm type early afternoon has an optimal and/or recommended time to conduct a physical activity between 12 pm and 3 pm.

In one embodiment, the circadian rhythm type late afternoon has an optimal and/or recommended time to conduct a physical activity between 3 pm and 6 pm.

In one embodiment, the circadian rhythm type early evening has an optimal and/or recommended time to conduct a physical activity between 6 pm and 9 pm.

In one embodiment, the circadian rhythm type late evening has an optimal and/or recommended time to conduct a physical activity between 9 pm and 12 am.

In one embodiment, the circadian rhythm type early night has an optimal and/or recommended time to conduct a physical activity between 12 am and 3 am.

In one embodiment, the circadian rhythm type late night has an optimal and/or recommended time to conduct a physical activity between 3 am and 6 am.

In one embodiment, the method can be used for calculating the prediction of circadian rhythm related disorder and the prediction of the effect of therapeutic interventions on the subject's circadian phase shift and amplitude.

In one embodiment, the method can be used for calculating the prediction of circadian rhythm related disorder and the prediction of the effect of a change in physical activity times on the subject's circadian phase shift and amplitude.

In one embodiment, the therapeutic intervention and/or modification of physical activity times are appropriate for a subject if it produces an outcome that in and of itself helps prevent, treat, and/or cure a disease.

In one embodiment, the method is used to prevent, ascertain, prognose or treat a circadian rhythm related disorder or to detect a perturbation of a normal biological state of the amplitude or circadian phase from the subject.

In some embodiments, the determined circadian rhythm, preferably the determined phase of the circadian rhythm, provides recommendations for physical activity of the subject, comprising one or more of recommended sleeping times, working times, mealtimes, sports times, intellectual task times, light exposure times, medical intervention times and/or medical diagnosis times.

In some embodiments, the method comprises calculating the amplitude and the phase of the circadian rhythm of the subject using a biomathematical model, preferably ZeitZeiger, Lasso and/or partial least square (PLS) , comprising:
a) Inputting the gene expression levels of the human subject quantified,
b) Loading at least one training data set, wherein said training data set comprises data entries of reference gene expression levels (from other subjects), comprising gene expression levels from circadian phase genes and amplitude prediction genes, wherein each data entry comprises a gene label, a weight factor, and an amplitude score and/or time parameters,
c) Determining a circadian phase value for the subject by applying the weight factor of each circadian phase gene from the data entries in step b) to each circadian phase gene expression level inputted in step a),
d) Determining an amplitude value for the subject by applying the weight factor of amplitude prediction genes from the data entries in step b) to each amplitude prediction gene expression level inputted in step a),
e) Computing the phase of the circadian rhythm by applying a time point of sample collection for the sample to the phase value from step c) and matching the phase value to the time parameters of the training data set from step b), and
f) Computing the amplitude of the circadian rhythm by matching the amplitude value from step c) with the amplitude score of the training data set from step b).

In one embodiment, reference data are primary data for all inputs, parameters, RNA quantities, model variables (dependent or independent), even from different sample types and in different algorithms. In some embodiments, reference data comprise data from samples at physiological state and/or data from samples at pathological state. Reference data sets are in some embodiments stored in databases.

In some embodiments, the computing step comprises
- Determining a circadian phase value based on the expression level of genes from the group of circadian phase genes;
- Determining an amplitude value based on the expression level of genes from the group of amplitude prediction genes.

In some embodiments, the calculation in steps c-f using the biomathematical model is executed by using the RNA expression levels from the sample of the subject determined as described herein as input values (step a), loading a training data set, and applying an algorithm to the input values and training data set, thereby generating an intermediate value and an output value.

In some embodiments, the intermediate value is generated in step c and d by the algorithm, wherein said intermediate value comprises the circadian phase value and amplitude value.

In some embodiments, intermediate values are further processed by the algorithm in step e and f for computing the output values.

In some embodiments, output values generated using the biomathematical model comprise the circadian rhythm and the amplitude.

As shown in the Examples, each circadian phase gene oscillates differently over a 24-hour period. Thus, each possible circadian rhythm has a unique combination of circadian phase gene expression and amplitude prediction gene expression. The ZeitZeiger algorithm calculates for each sample a circadian phase value and computes the circadian rhythm and amplitude by matching said values to training data set of the biomathematical model having the most similar combination.

In one embodiment, the algorithm is the ZeitZeiger algorithm or a modification of the ZeitZeiger algorithm.

In one embodiment, the algorithm is the LASSO algorithm or a modification of the of the LASSO algorithm.

In one embodiment, the algorithm is the Molecular-timetable algorithm or a modification of the Molecular-timetable algorithm.

In one embodiment, the algorithm is the partial least square algorithm or a modification of the partial least square algorithm.

As shown in the Examples, in non-limiting terms, optimal parameters for biomathematical model generation using an algorithm described herein, were determined by performing LOPO and/or LOSO.

As described in Example 2, the amplitude score and weighting factors were derived from the training data during the learning of the biomathematical model. In one embodiment, the amplitude score is the mean amplitude of the 5, 6, 7, 8, 9, 10 or more of the strongest genes. The amplitude can also be determined by the expression strength of certain genes.

In preferred embodiments, a combination of the ZeitZeiger algorithm, the PLS algorithm, and the LASSO algorithm is used. In one embodiment, a combination of the ZeitZeiger algorithm, and the LASSO algorithm is used. In one embodiment, a combination of the PLS algorithm and the LASSO algorithm is used. In one embodiment, a combination of the ZeitZeiger algorithm and the PLS algorithm is used.

In some embodiments, one, two, three or all said algorithms are modified. A description of the algorithm used is given below

In some embodiments, values obtained for circadian phase corresponds to circadian rhythm expressed as hours past DLMO (hours past dim-light melatonin onset).

The skilled person could not have expected that the achieved accuracy in the examples exceeds the accuracy of the DLMO measurement in the prior art. It is surprising that at least two or all of the found genes of the circadian phase gene group are suitable for the determination of the circadian rhythm.

In some embodiments, the output value comprises the amplitude that corresponds to a strength of a circadian rhythm.

In some embodiments, a gene from the circadian phase early-day gene group is selected from an early morning gene group and/or late morning gene group. In some embodiments, an early morning gene group comprises one or more genes selected from TEF, AC016727.1, TLR5, PDK1, HLF, PER3, PER2, ZBTB42, PER1, POLR2J4, SLC6A6, DUBR, BORCS6, SPRR2B and/or CRY2. In some embodiments, a late morning gene group comprises one or more genes selected from PIF1, TRIM35, LRAT, HERC3, ALDH3A1 and/or RPL23AP7.

In one embodiment, two or more genes from the circadian phase early-day gene group can be measured to determine the circadian phase. In one embodiment, all genes from the circadian phase early-day gene group can be measured to determine the circadian phase.

In one embodiment, two or more genes from the circadian phase early morning gene group can be measured to determine the circadian phase. In one embodiment, all genes from the circadian phase early morning gene group can be measured to determine the circadian phase.

In one embodiment, two or more genes from the circadian phase late morning gene group can be measured to determine the circadian phase. In one embodiment, all genes from the circadian phase late morning gene group can be measured to determine the circadian phase. In some embodiments, a gene from the circadian phase day gene group is selected from an early afternoon gene group and/or late afternoon gene group. In some embodiments, an early afternoon gene group comprises one or more genes selected from TGM5, TNS1, RORC, KLRG2, CRY1, and/or B3GNT2. In some embodiments, a late afternoon gene group comprises one or more genes selected from MINOS1, ANO7L1, LRRC37A3, PSMG3-AS1, AC011476.3, SPRR2A, KRT15, CAMKMT, GNB1L, SERPINE2, SAP30, and/or PLEKHF1.

In one embodiment, two or more genes from the circadian phase day gene group can be measured to determine the circadian phase. In one embodiment, all genes from the circadian phase day gene group can be measured to determine the circadian phase.

In one embodiment, two or more genes from the circadian phase an early afternoon gene group can be measured to determine the circadian phase. In one embodiment, all genes from the circadian phase an early afternoon gene group can be measured to determine the circadian phase.

In one embodiment, two or more genes from the circadian phase a late afternoon gene group can be measured to determine the circadian phase. In one embodiment, all genes from the circadian phase a late afternoon gene group can be measured to determine the circadian phase.

In some embodiments, a gene from the circadian phase evening gene group is selected from an early evening gene group and/or late evening gene group. In some embodiments, an early evening gene group comprises one or more genes selected from PGF, ARNTL, ARSJ, NPAS2, SPRY2 and/or URB1-AS1. In some embodiments, a late evening gene group comprises one or more genes selected from ZNF510, CLEC11A, GL12, NDUFAF4, RBM18, ZNF669, DlO3OS, PIBF1, CDPF1, P4HA1, PRR34-AS1 and/or ZNF749.

In one embodiment, two or more genes from the circadian phase evening gene group can be measured to determine the circadian phase. In one embodiment, all genes from the circadian phase evening gene group can be measured to determine the circadian phase.

In one embodiment, two or more genes from the circadian phase an early evening gene group can be measured to determine the circadian phase. In one embodiment, all genes from the circadian phase an early evening gene group can be measured to determine the circadian phase.

In one embodiment, two or more genes from the circadian phase a late evening gene group can be measured to determine the circadian phase. In one embodiment, all genes from the circadian phase a late evening gene group can be measured to determine the circadian phase.

In some embodiments, a gene from the circadian phase night gene group is selected from an early night gene group and/or a late night gene group. In some embodiments, an early night gene group comprises one or more genes selected from RRAD, POLR3GL, SLC22A15, NR1H3, AC048341.2, STXBP4, CD34, MAP3K14, NME7, SCGB2A2, LAYN, C1orf123, RAI14, FBLN7, PDE6B, GOT1, PDSS1, POLR2I and/or C12orf66. In some embodiments, a late night gene group comprises one or more genes selected from NR1D1, CDC25B, ZSCAN31, VWF, TDRKH, DBP, AL391121.1, MOSPD2, CNN1, CDCA3, NR1D2, CIART, ZNF296 and/or U2AF1.

In one embodiment, two or more genes from the circadian phase night gene group can be measured to determine the circadian phase. In one embodiment, all genes from the circadian phase night gene group can be measured to determine the circadian phase.

In one embodiment, two or more genes from the circadian phase an early night gene group can be measured to determine the circadian phase. In one embodiment, all genes from the circadian phase an early night gene group can be measured to determine the circadian phase.

In one embodiment, two or more genes from the circadian phase a late night gene group can be measured to determine the circadian phase. In one embodiment, all genes from the circadian phase a late night gene group can be measured to determine the circadian phase.

In some embodiments, an amplitude prediction gene is selected from one or more of DUBR, GL12, MOSPD2, SPRY2, TLR5, ZNF296, ZNF749 and/or HERC3.

In one embodiment, 2, 3, 4, 5, 6, 7, 8, 9 or 10 amplitude prediction genes can be measured to determine the amplitude.

In some embodiments, the determined amplitude and/or circadian phase indicate means to prevent and/or treat a circadian rhythm related disorder, comprising one or more of a disturbance in health or mental conditions, for example an advanced or delayed sleep-wake phase disorder, a non-24-hour sleep-wake rhythm disorder, a shift work disorder, and/or a jet lag disorder.

In embodiments, chronotherapy may comprise prevention and/or treatment a circadian rhythm related disorder of a subject.

In one embodiment, the circadian rhythm and the amplitude can be determined during the chronotherapy for evaluating the effectiveness of the chronotherapy.

In one embodiment, the circadian rhythm and the amplitude can be determined before chronotherapy for selecting a type of treatment.

In one embodiment, the circadian rhythm and the amplitude can be determined after the chronotherapy for evaluating effectiveness of the chronotherapy, wherein said effectiveness comprises an improved circadian phase and amplitude, improved circadian rhythm related disorder, improved gene expression of circadian phase genes and amplitude prediction genes at levels matching the circadian phase and circadian rhythm type, improved gene expression of amplitude prediction genes at levels of a strong amplitude as compared before chronotherapy.

In some embodiments, the RNA expression is determined using a method selected from the group consisting of NanoString, quantitative PCR, microarray, or sequencing, preferably NanoString.

Several RNA quantification methods are known in the prior art. In one embodiment, NanoString technology is preferably used for quantifying RNA expression in the sample from the subject. In one embodiment, an absolute quantification of RNA expression is used for determining absolute RNA quantities. In one embodiment, a relative quantification of RNA expression is used for determining RNA quantities.

The invention further relates to a method for providing optimal and/or recommended physical activity times for a subject comprising carrying out the method described herein, wherein said physical activity times comprise one or more of recommended sleeping times, go to bed times, working times, meal times, coffee or tea drinking times, sport times, intellectual task times, creative task times, light exposure times, medical intervention times and/or medical diagnosis times. In embodiments, chronotherapy comprises adjusting physical activities of a subject to circadian rhythm type of that subject.

The present invention further provides a diagnostic kit including an inlet for at least one hair root sample provided in a substrate, wherein numerals, letters or symbols are displayed on the sides of the inlet, and the samples are placed at one time point or at intervals of 2 to 6 hours for 24 to 48 hours.

The invention also relates to a computer readable storage medium comprising instructions to configure a processor to perform a method described herein.

The invention further relates to a computer-readable storage medium according to the preceding claim, comprising instructions to configure a processor to perform a method, comprising:
- a biomathematical model with quantitative RNA expression values as input values determined in a method according to any one of the preceding claims, and
- a training data set comprising data entries of reference gene expression levels (from other subjects), comprising gene expression levels from circadian phase genes and amplitude prediction genes, wherein each data entry comprises a gene label, a weight factor, and an amplitude score and/or time parameters.

In some embodiments, a processor-readable medium is provided comprising code representing instructions for enabling a processor to use in one or more mathematical models one or more parameters related to determining the circadian phase and amplitude for a sample of a subject.

In one embodiment, data is input into the mathematical model comprising an RNA expression level of a sample and a time of sampling of the subject, and a training data set is loaded, comprising a model parameter, weight factor, amplitude score, and/or reference data. In one embodiment, executing the algorithm for biomathematical modelling comprise parameterizing and updating the models to the sample of the subject, time of sample and genes determined for the circadian phase and the amplitude so that the updating uses said data; so that the algorithms enable determination of the circadian rhythm and the amplitude of the subject.

In one embodiment, an output of the algorithm comprises the phase and amplitude of a circadian rhythm of a subject at time of sampling.

Further examples of the computation-based method for determining circadian phase of the circadian rhythm and amplitude are described in detail below.

### DETAILED DESCRIPTION OF THE INVENTION

The present invention relates to a method for determining a circadian rhythm of a human subject based on the RNA expression levels quantified in samples from the human subject.

All words and terms used herein shall have the same meaning commonly given to them by the person skilled in the art, unless the context indicates a different meaning. All terms used in the singular shall include the plural of that term and vice versa

In the context of the present specification, the term "external time" relates to the time of day as indicated by a conventional 24-hour clock.

### Circadian rhythm, circadian phase, amplitude

As used herein, a "circadian rhythm" occurs over approximately one day, i.e., a 24-hour period, and may also be referred to or understood as an endogenous biological rhythm, endogenous oscillation, individual circadian clock, or internal clock. By way of example, the circadian rhythm refers to the cycle of human oscillatory gene expression, human hormone secretion, neurotransmitter secretion cycle, core body temperature rhythm to regulate physical activity times including rest times. Such oscillation is adjusted to the local environment by external cues including light, food and temperature.

The circadian rhythm ensures that human biological rhythms, including sleep, repeat in 22- to 25-hour units. These rhythms temporally structure physiology and behavior to make optimal use of the fourth dimension (i.e., time) for fitness, e.g., by anticipating predictable changes in environmental conditions and by temporally merging compatible processes or temporally separating incompatible processes.

For example, the dark hormone melatonin is secreted by the pineal gland, and is typically secreted a few hours before habitual bed time and its secretion is suppressed by light. Cortisol, secreted by the adrenal gland, causes preparation for waking and typically peaks briefly before waking.

The "circadian phase" reflects the period of a circadian rhythm where a peak and trough occur, e.g., peak and trough of performance within the 24 h. A phase shift in the circadian rhythm means shifts in physical activity including rest, e.g. bedtime and wake-up time occurs earlier in the day (phase advance) or later in the day (phase delay). The phase response curve (PRC) is a curve that describes the relationship between a stimulus, such as light exposure, and a response, in this case a shift in circadian rhythm (phase shift). The PRC is important because it can determine when to properly adjust light and melatonin to advance (or delay) the circadian phase.

In some embodiments, the determined circadian rhythm, preferably the determined phase of the circadian rhythm, provides recommendations for physical activity, including sleep of the subject. In some embodiments, physical activity times comprise one or more of recommended sleeping times, go to bed times, working times, mealtimes, coffee or tea drinking times, sport times, intellectual task times, creative task times, light exposure times, medical intervention times and/or medical diagnosis times. In some embodiment, rest times comprise one or more of recommended times for sleeping, breaks, naps, and/or relaxing.

The term "circadian oscillating gene" refers to genes that exhibit rhythmic transcription in a particular tissue, cells in a body fluid, or other gene-containing cells of the body depending on the time of day. This applies to approximately 10% of all expressed genes. Genes that belong to the core of the circadian oscillator (so-called clock genes) are rhythmically active in almost all tissues or gene-containing cells of the body. However, the majority of circadian oscillating genes (the so-called clock controlled genes) exhibit cell type-specific rhythmic transcription. When subjects are well synchronized with the external light-dark cycle, the phase of peripheral gene expression correlates with the phase of hormonal rhythms controlled by the central clock in the suprachiasmatic nucleus (SCN).

It is known to those skilled in the art that the phase and amplitude of peripheral clocks vary between different tissues and organs and also differ from the phase and amplitude in the SCN. In addition, peripheral clocks may coordinate gene expression locally. Each cell may have a circadian clockwork that is activated by the right environment, humoral or neural cue. For example, a vascular clock may generate an amplified and synchronized vascular rhythm in response to uncoordinated blood-borne signals from central and peripheral clocks. These signals may be the diurnal release of catecholamines and steroids or the periodic bioavailability of signals such as vitamins associated with metabolic cycles.

The existence of multiple oscillating genes is a common feature of all circadian systems in multicellular organisms described to date. In a system in which the constituent rhythms have distinct phases, this may be the most effective way to generate the complex phase relationships among multiple higher-order rhythms that are known to exist and are almost certainly critical for normal function. Peripheral oscillators allow biological rhythms to be easily tuned to small, gradual changes in the phase of the input signal. However, when the phase change is completely disruptive, e.g., due to distorted environmental cues such as cross-time zone travel, the switch from a daytime work schedule to nighttime work schedule, or a large hormone release such as sympatho-adrenal activation under severe stress conditions, the phase relationships between SCNs and peripheral clocks and also among various peripheral clocks are abolished, resulting in a transiently highly disorganized circadian system. Therefore, one aspect of the present invention provides a method for modulating the core clock oscillator by modulating a peripheral clock. In a preferred embodiment, the peripheral clock is that of the hair root.

It is to be understood by one skilled in the art that the phase and amplitude of peripheral clocks vary between different tissues and organs, in addition to differing from the phase and amplitude in the SCN.

The term "amplitude" of the rhythm describes the difference in the level between peak and trough values and is optimal at 100%, 99%, 98%, 97%, 96%, 95%, 94%, 93%, 92%, 91% or 90% of the maximum difference. The amplitude refers to the strength of a circadian oscillator. Therefore, the strength of a person's circadian rhythm can also be determined by the expression level of amplitude prediction genes as a measurement of the amplitude of a circadian oscillatory variable.

For example, such circadian oscillatory variables include the expression level of a circadian oscillatory gene or the expression level of a set of circadian oscillatory genes, hormone levels (e.g., cortisol), core body temperature, or the behavioral activity level determined by actigraphy. In actigraphy, a person's activity profile is determined over several days using wearable devices. A large amplitude of a circadian oscillation variable is indicative of a strong individual circadian rhythm, and a small amplitude is indicative of a weak circadian rhythm.

The circadian phase and the amplitude can be determined at a single time point or more than one time point.

The present invention uses quantitative analysis of RNA expression level of genes also defined as circadian oscillatory genes, also termed as "circadian phase genes". For each oscillatory circadian phase gene, the peak of expression corresponds to specific times over the 24-hour day and these genes are grouped into early day gene, day gene, evening gene, and night gene groups. In one embodiment, the oscillation of circadian phase gene transcripts has its maximum at time or time period of the day.

### Early day gene

The early day gene group can be further sub-grouped into early morning gene group comprising the daytime from 6 am to 9 am and late morning gene group comprising the daytime from 9 am to-12 pm.

In embodiments, an early morning gene can be selected from TEF, AC016727.1, TLR5, PDK1, HLF, PER3, PER2, ZBTB42, PER1, POLR2J4, SLC6A6, DUBR, BORCS6, SPRR2B and/or CRY2.

In embodiments, a late morning gene can be selected from PIF1, TRIM35, LRAT, HERC3, ALDH3A1 and/or RPL23AP7.

### Day gene

The day gene group can be further sub-grouped into early afternoon gene group comprising the daytime from 12 pm to 3 pm and late afternoon gene group comprising the daytime from 3 pm to 6 pm, and, in embodiments, comprises one or more genes of:
- An early afternoon gene selected from TGM5, TNS1, RORC, KLRG2, CRY1, and/or B3GNT2, and/or
- A late afternoon gene selected from MINOS1, ANO7L1, LRRC37A3, PSMG3-AS1, AC011476.3, SPRR2A, KRT15, CAMKMT, GNB1L, SERPINE2, SAP30, and/or PLEKHF1.

### Evening gene

The evening gene group can be further sub-grouped into early evening gene group comprising the daytime from 6 pm to 9 pm and late evening gene group comprising the daytime from 9 pm to 12 am, and, in embodiments, comprises one or more genes of:
- An early evening gene selected from PGF, ARNTL, ARSJ, NPAS2, SPRY2 and/or URB1-AS1, and/or
- A late evening gene selected from ZNF510, CLEC11A, GLI2, NDUFAF4, RBM18, ZNF669, DlO3OS, PIBF1, CDPF1, P4HA1, PRR34-AS1 and/or ZNF749

### Night gene

The night gene group can be further sub-grouped into early night gene group comprising the daytime from 12 am to 3 am and late night gene group 3 am to 6 am, and, in embodiments, comprises one or more genes of:
- An early night gene selected from RRAD, POLR3GL, SLC22A15, NR1H3, AC048341.2, STXBP4, CD34, MAP3K14, NME7, SCGB2A2, LAYN, C1orf123, RAI14, FBLN7, PDE6B, GOT1, PDSS1, POLR2I and/or C12orf66, and/or,
- Late night gene selected from NR1D1, CDC25B, ZSCAN31, VWF, TDRKH, DBP, AL391121.1, MOSPD2, CNN1, CDCA3, NR1D2, CIART, ZNF296 and/or U2AF1.

### Amplitude prediction gene

As used herein, "amplitude prediction genes" refer to genes showing a correlation of their expression with an amplitude of the circadian rhythm of the subject. Preferably, amplitude prediction genes are not rhythmic. By way of example, specific genes those RNA expression level can be used to predict the amplitude of a circadian rhythm from a single sample (i.e. without measuring the time series) have been found. In embodiments, the amplitude prediction gene is selected from one or more of DUBR, GL12, MOSPD2, SPRY2, TLR5, ZNF296, ZNF749 and/or HERC3.

A biomathematical algorithm, such as LASSO, ZeitZeiger, Partial Least Square, can be used to determine optimal genes and the corresponding parameters for amplitude prediction. For defining the amplitude, amplitude score as mean amplitude of the top5 or top10 rhythmic genes is introduced into the biomathematical model. Other genes, including predictor genes for phase, also correlate with amplitude, which may affect correct phase prediction. A correction factor corrects according to amplitude (only for subjects with amplitudes <2, slope as correction factor). For example, for subjects with low amplitude, amplitude correction improves model performance substantially.

### Circadian rhythm related disorders, lifestyle, physical activity

The terms "disorder" or "disease", as used herein, can be used interchangeably.

Circadian rhythm related disorders are associated with changes in strength of circadian rhythm, amplitude, circadian rhythm phase shift, and internal desynchronization. It can be caused by endogenous factors, such as a genetic variation, age, gender, menorrhea, disturbed melatonin or cortisol secretion, delayed or advanced sleep phase syndrome, or external factors, such as environmental factors, e.g., time zone crossing travel, working hours at night, rotating shift work, stress, Jetlag, social jetlag. Circadian rhythm disorders comprise Non-24-Hour disorder, circadian rhythm sleep disorder types comprising jet lag type (jet lag disorder), shift work type (shift work disorder), altered sleep phase types including delayed sleep phase syndrome and advanced sleep phase syndrome. Circadian sleep rhythm disturbances may also occur in patients with other diseases including, but not limited to, neurodegenerative diseases such Alzheimer's or Parkinson's disease, patients who have had head trauma or encephalitis, patients with psychiatric diseases, and patients at intensive care units.

Circadian rhythm sleep disorder refer to desynchronization between internal sleep-wake rhythms and the light-darkness cycle. Patients typically suffer from insomnia, excessive daytime sleepiness, or both, which typically resolve as the body clock realigns itself. If the rhythms adapt back, the symptoms may subside over several days or, in some patients (e.g., elderly patients), over several weeks or months.

As known to the skilled person, light is a strong synchronizer of circadian rhythms. As a therapeutic measure, exposure to bright light (sunlight or artificial light with an intensity of 5,000 to 10,000 lux) and activity after the desired wake-up time and the use of sunglasses to reduce light exposure before the desired bedtime is beneficial for rapid adaptation to the circadian rhythm. An important factor here is the correct determination of the circadian phase of the circadian rhythm and amplitude.

The present invention also provides means and methods for predicting circadian rhythm or circadian rhythm phase shift disturbances caused by an internal clock disturbance, such as circadian rhythm sleep disturbance, and more particularly, to provide a method for accurate and simple diagnosis of circadian rhythm disturbance.

In one embodiment, the determined circadian rhythm phase and amplitude of the circadian rhythm of a patient is compared to a non-disturbed circadian rhythm.

In embodiments, the circadian rhythm of the human subject is determined as one or more circadian rhythm types with a defined circadian phase of optimal and/or recommended physical activity of the subject. "Optimal" as used herein, refers to the most desired performance of a physical activity, as described herein, at the defined circadian phase. "Recommended," as used here, refers to the suggestion of physical activity during a defined circadian phase at which the body is ready or otherwise optimized for it. As known in the art, body readiness for a physical activity is enhancing the performance and health of the subject.

"Physical activity", as used herein, is understood as any action or biological state of or with the body of a human subject. Physical activity comprise one or more of going to bed, work, meal intake, sport, intellectual task, free time, light exposure, medical intervention and/or medical diagnostics. The present invention enables recommendations for optimal time for any given physical activity.

The "lifestyle", as used herein, refers to activities of the subject at certain times of the 24-hour period. Lifestyle comprises, without limitation, sleeping times, go to bed times, creative task times, working times, mealtimes, coffee or tea drinking times, sports times, intellectual task times, light exposure times, medical diagnosis times, and medical intervention times in relation to a circadian phase. In one embodiment, a subject's lifestyle is assessed for the time of sampling. In one embodiment, subject's lifestyle is graded for the time of sampling.

The present invention relates to a method for providing information of circadian rhythms, comprising the step of checking an endogenous factor at least one time within 24h, in which a circadian rhythm can be determined by determining an amplitude and a phase variation of a circadian rhythm. The present invention relates to means and methods for providing information to a subject about its circadian rhythm and strength of circadian rhythm. In one embodiment, one or more endogenous factors in a subject is measured preferably at a single time point within 24h. In one embodiment, the change of one or more endogenous factors in a subject is preferably determined in a single sample of a subject taken within 24h.

When caused externally, other circadian body rhythms, including temperature and hormone secretion, can become out of synchronization with the light-dark cycle (external desynchronization) and with each other (internal desynchronization); in addition to insomnia and excessive sleepiness, these changes can cause forgetfulness, behavioral changes, fatigue, weakness, nausea, malaise, irritability, and/or mental health disorders (i.e. depression). The risk of cancer, neurological diseases, immune deficiencies, stress induced mental and physical disturbance, cardiovascular and metabolic disorders may also be increased.

As used herein, the terms "cardiovascular disorder", "heart disease", "cardiac disease" are interchangeable with, or at least related to, the terms "cardiomyopathy" or "cardiovascular related disorders". Cardiovascular disorders are a large class of diseases that affect the heart and/or blood vessels (arteries and veins). Cardiovascular disorders include arrhythmias, vascular disease, myocardial infarction, heart failure, myocarditis, atherosclerosis, restenosis, coronary heart disease, coronary artery disease, atherosclerotic cardiovascular disease, arterial hypertension, cardiac fibrosis, stroke, sudden cardiac death syndrome, heart failure, ischemic heart disease, ischemic cardiomyopathy, myocardial infarction, coronary artery calcification. These diseases have similar causes, mechanisms, and treatments. Most cardiovascular disorders have common risk factors, including inflammation, fibrosis, diabetes, cholesterol, and vascular deposits.

"Metabolic disorder" is related to a disorder that negatively alters the body's processing and distribution of substrates, supplements and nutrients including proteins, carbohydrates, fatty acids, and lipids resulting in abnormal chemical reactions in the body which is associated with pathophysiological changes in the body.

The term "pathological state" and "diseased" are used interchangeably and comprise any change in health and well-being related to the circadian rhythm. In one embodiment, the phase of the circadian rhythm and the amplitude is determined for the human subject at diseased or pathological state. In one embodiment, the phase of the circadian rhythm and the amplitude is determined for the human subject at physiological state. The terms "physiological state" and "normal biological state" are used interchangeably and comprise RNA expression levels of circadian phase genes and amplitude predictions matching the phase and the amplitude of the circadian rhythm.

### Sample preparation. Quantification, normalization of RNA expression levels

"RNA expression" may also be referred to as "transcript expression", or determination of "transcriptomics". Quantitative transcriptomics requires the analysis of RNA expression levels in samples. A "provided" sample may have been obtained from another person and given to the person (or machine) performing the procedure. A "sample" (e.g., a test sample) from a subject means a sample obtained from a subject - preferably suspected of having a non-optimal lifestyle with respect to their circadian rhythm - or from a subject with a circadian rhythm related disorder, a disturbance in health, a disturbance in mental conditions, or a non-diseased subject ("control," "normal").

A "non-diseased subject", "control," or "normal" subject refers to a subject with non-disturbed circadian rhythm or a subject not suffering from a circadian rhythm related disorder, wherein the circadian rhythm determined by means and method described herein preferably matches the subject's internal clock, with normal amplitude as described herein. The amplitude of the circadian rhythm correlates with the level of gene expression of the amplitude prediction genes used for comparison. Many suitable sample types will be evident to a skilled person.

Methods for storing and lysing of samples and RNA extraction from said samples are well-known to a skilled person. A preferred method storing and lysing samples and RNA extraction from specific samples is provided in non-limiting terms in the Examples.

RNA quantities are a number, e.g. an integral number, a decimal number, of RNA determined by an appropriate RNA quantification method, as well-known to a skilled person, or obtained from a public database or obtained from published literature. Relative RNA expression levels are a number, e.g. an integral number, a decimal number, or a percentage value of RNA referring to an RNA expression level obtained from another sample determined by an appropriate RNA quantification method, known to a skilled person, or obtained from a public database or obtained from published literature. Examples for RNA with corresponding expression levels are provided herein.

In one embodiment, the RNA expression level of the sample is determined by a method, as provided in non-limiting terms by the Examples described herein, wherein the method comprises (i) solubilizing the sample, (ii) extracting RNA from solubilized sample of step (i) according to the RNA quantification method, (iii) transferring said extracted RNA from step (ii) to a device, preferably a NanoString, of said RNA quantification method identifying and quantifying the RNA expression level in said sample.

The RNA expression level of a gene, whether relative or absolute, may be determined through various methods suitable for quantification. A suitable quantification method may be selected from a group of methods such as quantitative PCR, NanoString, RNASeq, QuantiGene, and microarray, Luminex assay. In one embodiment, quantitative PCR is used to determine RNA expression levels. In one embodiment, NanoString technology is used to determine RNA expression levels. In one embodiment, RNASeq technology is used to determine RNA expression levels. The skilled person is able to choose the appropriate method without much added effort. In one embodiment, microarry technology is used to determine RNA expression levels.

Typically, when qPCR or NanoString technology is used to determine expression level, the normalized expression level is determined as the expression of a gene relative to the expression of a housekeeping gene or genes.

NanoString technology is a sensitive method for determining gene expression levels. It uses probes that hybridize to RNA molecules in solution. Each target-specific probe corresponding to a transcript of interest carries a so-called "molecular barcode" that allows its unique identification and quantification by microscopic single-molecule imaging.

Based on the fact that about 10% of all genes in almost all human cells are transcribed rhythmically, the principle of the invention is that the phase of peak expression is gene-dependent. The relative concentrations of oscillating transcripts are therefore unique at each time of day.

A "housekeeping gene", as used herein, refers to a gene that is expressed independently of cell type, cell stage, patho-physiological conditions, time-of-day and external influences, in contrast to regulated genes. In one embodiment, in said RNA quantification method, a housekeeping gene is also quantitatively determined and used for the normalization of the amount of cellular RNA in the sample of said subject. The housekeeping gene is preferably selected from the group consisting of GAPDH, PGK1, CLTC, PSMB2, ACTB (beta-Actin), 18s rRNA, PPIA, UBC, EIF4E2 and HPRT1, or any other housekeeping gene known in the art.

The properties and nucleotide sequences of the RNA determined in the sample of the subject are well-known and can be determined routinely, as well as downloaded from various known databases. See. e.g., the database at the world wide web site https://www.ncbi.nlm.nih.gov/nuccore. Information to some of the RNA discussed herein, is provided in the Examples.

A "significant" difference in a value, as used herein, can refer to a difference which is reproducible or statistically significant, as determined using statistical methods that are appropriate and known in the art, generally with a probability value of less than five percent chance of the change being due to random variation. In general, a statistically significant value is at least two standard deviations from the value in a "normal" control subject or reference. Suitable statistical tests will be evident to a skilled worker. For example, a significant difference in the amount of a protein compared to a baseline value can be about 50% less, or 2-fold higher.

It is generally not practical in a clinical or research setting to use patient samples as sources for baseline controls. Therefore, one can use any of variety of references as described herein. Those skilled in the art are aware that a baseline or normal value need not be established for each method for determining RNA expression level when it is performed, but that reference or

normal values may be established by reference to some form of stored information about a previously determined reference value for a particular RNA or panel of RNA, such as a reference value established by one of the methods described. Such a form of stored information may include, for example, a reference table, a listing or electronic file of population or individual data relating to "normal values" (control) or positive controls, a medical record for the patient in which data from previous evaluations are recorded, a receiver operator characteristic (ROC) curve, or any other source of data relating to reference values that is useful to the patient. In an embodiment in which the change of adapting the individual lifestyle to fit one's own circadian rhythm is monitored, a reference value may be based on previous measurements of the same subject before the treatment was administered.

An essential step in an RNA expression determination is normalization. "Normalization" refers to calculation step, in which the raw data are adjusted for factors that prevent direct comparison of expression measures. Errors in normalization can have a significant impact on downstream analysis, such as inflated false positives in differential expression analysis. Several normal normalization methods are known in the prior art, including normalization by control genes, e.g. housekeeping genes, library size or by expression means of determined RNA expression levels (Evans C, Hardin J, Stoebel DM. Selecting between-sample RNA-Seq normalization methods from the perspective of their assumptions. Brief Bioinform. 2018; 19(5):776-792).

### Sample prerapation

The test kit for sampling preferably comprises a sample extractor, a sample tube comprising a buffer solution for storing hair follicle samples, a label, an instruction for use, and an envelope for sending back the sample. The hair root sample is stored in an "RNA Later"-like buffer solution. Extraction of total RNA from hair follicles using TRIZol as described in the Examples. RNA quantification using the Qubit RNA BR Assay in the Qubit 4 Fluorometer is performed following the manual instructions. RNA hybridization and the generation of raw expression data using NanoString Technologies and calculating of the internal time from hair follicle gene expression data using the BodyTime App are described in Examples.

### Subject, treatment, therapy

In one aspect of the present invention there is provided an circadian rhythm of a subject according to the invention as herein described for use in the treatment of a medical condition associated with changes in circadian rhythm, wherein the medical condition associated with changes in circadian rhythm can be a circadian rhythm related disorder comprising advanced sleep-wake phase disorder, delayed sleep-wake phase disorder, irregular sleep-wake rhythm disorder, Jet lag disorder, Non-24-hour sleep-wake rhythm disorder, metabolic disorders or shift work disorder. Symptoms of a circadian rhythm related disorder comprise for example daytime sleepiness, insomnia, tiredness, decreased alertness, and problems with memory and decision-making.

As used herein, the "patient" or "subject" refers to a human, preferably a patient having a disturbed circadian rhythm, but can also be any other mammal, such as a domestic animal (e.g. a dog, cat or the like), a farm animal (e.g. a cow, sheep, pig, horse or the like) or a laboratory animal (e.g. a monkey, rat, mouse, rabbit, guinea pig or the like). The term "patient" preferably refers to a "subject" suffering from or suspected of suffering from a circadian rhythm related disorder and/or changes in circadian rhythm.

In the present invention "treatment" or "therapy" generally means to obtain a desired pharmacological effect and/or physiological effect. The effect may be prophylactic in view of completely or partially preventing a disease and/or a symptom, for example by reducing the risk of a subject having a disease or symptom or may be therapeutic in view of partially or completely curing a disease and/or adverse effect of the disease. In the present invention, "assess" also means to discover circadian rhythm type, circadian rhythm related disorder and/or changes in circadian rhythm. In the present invention, the circadian rhythm and the amplitude for "grading" of subjects lifestyle in relation to the determined circadian rhythm comprise to predict occurrence, presence, cause or course of a disease or to predict the effect of a treatment, wherein treatment comprises changes in subjects lifestyle according to subject's circadian rhythm,. The term "evaluating", as used herein, usually means determining whether expected outcomes were met and comprise measuring effectiveness of a medical care, a treatment, a nutritional intervention, and a physical activity.

In the present invention, "therapy" includes arbitrary treatments of diseases or conditions in mammals, in particular, humans, for example, the following treatments (a) to (c): (a) Prevention of onset of a disease, condition or symptom in a patient; (b) Inhibition of a symptom of a condition, that is, prevention of progression of the symptom; (c) Amelioration of a symptom of a condition, that is, induction of regression of the disease or symptom.

The phrase "therapeutically effective" is intended to include, within the scope of sound medical judgment, excessive toxicity, irritation, and/or other problems or complications, but commensurate with a reasonable benefit/risk ratio. As used herein to refer to the therapeutic invention, nutritional intervention, activity suitable for a subject that produces a result that in and of itself helps to prevent, to treat and/or to cure a disease. These include risk factor reduction (e.g., diet, exercise, stress reduction), pharmacologic therapy (drugs), acupuncture, and interventional therapy.

In one embodiment, the subject that has taken a sample and provided it for circadian rhythm determination receives information about its circadian rhythm, circadian phases, and strength of its circadian rhythm. In one embodiment, the subject that has taken a sample and provided it for circadian rhythm determination receives information about its optimal circadian rhythm.

In one embodiment, the subject who has taken a sample and provided it for circadian rhythm determination is provided with information comprising optimal and/or recommended physical activity times for a subject, wherein said physical activity times comprise one or more of recommended sleeping times, going to bed times, working times, mealtimes, coffee or tea drinking times, sport times, intellectual task times, creative task times, light exposure times, medical intervention times, and/or medical diagnosis times. In one embodiment, the optimal and/or recommended physical activity times for a subject is therapeutically effective for a treatment of a circadian rhythm related disorder. The present invention therefore provides means for treating a circadian rhythm related disorder as described herein.

In one embodiment, the subject is provided the information electronically, such as via email, short message service, an instant messaging service, or via an application on the Internet or mobile phone. In one embodiment, the information is provided to the subject in writing, such as printed out and sent by letter.

In some embodiments, said patient has symptoms of reduced gene expression at RNA level involved in circadian rhythm related disorders or symptoms of increased gene expression of at RNA level involved in circadian rhythm related disorders.

In some embodiments, said patient has no symptoms of reduced gene expression at RNA level involved in circadian rhythm related disorders or no symptoms of increased gene expression of at RNA level involved in circadian rhythm related disorders.

In some embodiments, said patient at risk of developing symptoms of reduced gene expression at RNA level involved in circadian rhythm related disorders or at risk of developing symptoms of increased gene expression of at RNA level involved in circadian rhythm related disorders.

### Gene expression, nucleic acid

A "gene" refers to a DNA region that encodes a gene product including regions regulating the production of the gene product, regardless of whether these sequences are adjacent to coding and/or transcribed sequences. A gene comprises, but is not limited to, promoter sequences, terminators, translational regulatory sequences such as ribosome binding sites and internal ribosome entry sites, enhancers, silencers, insulators, boundary elements, replication origins, matrix attachment sites, and locus control regions.

"Gene expression" is particularly the conversion of the information encoded by a gene into a gene product. A gene product can be the direct transcription product of a gene (e.g., mRNA, tRNA, rRNA, antisense RNA, ribozyme, structural RNA or any other type of RNA) or a protein produced by translation of a mRNA. Gene products also include RNAs modified by processes such as capping, polyadenylation, methylation, and editing, as well as proteins modified by processes such as methylation, acetylation, phosphorylation, ubiquitination, ADP-ribosylation, myristoylation, and glycosylation. As used herein, "RNA" is used interchangeably with "mRNA" and "transcript".

As used herein, the terms "nucleic acids", "polynucleotide" or "nucleic acid molecule" relate to their commonly used meaning, and refer e.g. to messenger RNA (mRNA), RNA, genomic RNA (gRNA), plus strand RNA (RNA(+)), minus strand RNA (RNA(-)), genomic DNA (gDNA), complementary DNA (cDNA) or recombinant DNA. Polynucleotides include single and double stranded polynucleotides. Preferably, polynucleotides of the invention include polynucleotides or variants having at least about 50%, 55%, 60%, 65%, 70%, 75%, 80%, 85%, 90%, 95%, 96%, 97%, 98%, 99% or 100%) sequence identity to any of the reference sequences described herein, typically where the variant maintains at least one biological activity of the reference sequence.

### Zeitzeiger, Lasso. Partial least square

Several machine learning methods are useful to determine internal time from a subject sample, including, but not limited to, ZeitZeiger, LASSO, and PLS. " ZeitZeiger" is an algorithm for predicting a periodic variable (e.g., time of day) from a high-dimensional observation. ZeitZeiger learns a sparse representation of the variation associated with the periodic variable in the training observations, and then uses the maximum likelihood method to make a prediction for a test observationZeitZeiger is a method to predict the value of a periodic variable, which we define as being continuous and bounded, where the maximum value is equivalent to the minimum value. For simplicity, we denote the periodic variable here as 'time,' but ZeitZeiger can be applied to any type of periodic measurement. (Nucleic Acids Res. 2016 May 5; 44(8):e80m and Genome Medicine 2071; 9(1): 19). The ZeitZeiger vignette describes the bioinformatics workflow (https://github.com/hugheylab/zeitzeiger/blob/master/vignettes/introduction.Rmd).

"LASSO" refers to a least absolute shrinkage and selection operator algorithm for constructing a prediction model (Friedman J, Hastie T, Tibshirani R (2010). "Regularization Paths for Generalized Linear Models via Coordinate Descent." Journal of Statistical Software, 33(1), 1-22. https://www.jstatsoft.org/v33/i01/; Friedman J, Hastie T, Tibshirani R (2010) A note on the group lasso and a sparse group lasso. arXiv preprint arXiv:1001.0736). The glmnet R package under the "mgaussian" family can be used to find the optimal LASSO model. The calculation using LASSO comprises: (1) Making a prediction on the prediction dataset under the "mgaussian" family and receive predicted LASSO values; (2) Compute circular means and standard deviations for summarizing the model calculated.

"PLS" refers to partial least square algorithm for constructing a prediction model. The *pls* R package can be used to find the optimal PLS model (Boulesteix AL, Strimmer K. Partial least squares: a versatile tool for the analysis of high-dimensional genomic data. Briefings in Bioinformatics. 2007;8:32-44, Laing E E, et al. (2017) Blood transcriptome based biomarkers for human circadian phase. eLife 6:e20214; ). PLS vignette describes the bioinformatics workflow (https://cran.r-project.org/web/packages/pls/vignettes/pls-manual.pdf). The calculation using PLS comprises: (1) Make a prediction on the prediction dataset by using values received with LASSO and receiving pls.fit and pls.pred, (2) Compute circular means and standard deviations for summarizing the model calculated.

### Data entry. Input, gene label, amplitude score. Weight factor. Matching, parameter

For determining the circadian phase of a human subject, "individual parameter" or "parameter", as used herein, comprise one or more of sampling time, patient age, gender, lifestyle, eating behavior, physical activities hours, sleeping hours and/or time zone.

The "time variation of a parameter", as used herein, comprises a change of the input value in a time course that both shifts the output signal in time and changes other parameters and behavior.

The "amplitude score" refers to the mean amplitude of the top strongest genes correlating with the amplitude. In some embodiments, the mean expression of the top 5, 6, 7, 8, 9, 10 or more of the strongest genes is used to calculated the amplitude score.

The "weight factor" refers to a correction factor determined from the training data set, as described in the Examples. Calculating the weighting factor comprises (i) Determining amplitude by using trainig data, (ii) Identifying the preferable genes for amplitude prediction using machine learning (e.g., LASSO), (iii) 3 Analyzing the correlation of amplitudes with the expression of other genes (phase predictors), (iv) Correcting expression values by using the correlation of step (iii) using the slope and the mean amp (3.54) of the training data.

"Matching" refers to the level of correspondence of a value or determined state to a normal or control or non-disturbed value or state.

"Parameter" or "Model parameter" refers to a configuration variable that is internal to the model and whose value can be estimated from data. The determination of the parameter for the biomathematical model using an algorithm is described in the Examples.

### Computer program. Computer readable storage medium, instructions, processor

A further aspect of the invention relates to a computer program adapted to execute a mathematical modelling algorithm that will be performed by a computing device/module to produce outputs given data provided as inputs according to preceding claims, wherein said computer program is written in a programming language selected from a group comprising R, Fortran, C#, C/C++, High Level Shading Language, or Python.

In some embodiments, a "computer" or "computing device" may be used. Such a computer may be, for example, a mainframe computer, a desktop computer, a notebook or laptop computer, a portable device such as a data acquisition and storage device, or a processing device integrated into another device such as a scanner for tomography. In some cases, the computer may be a "dumb" terminal used to access data or processors over a network.

In some embodiments of the present invention, the mathematical model comprises processor-readable media comprising: one or more parameters relating to the sample and/or the subject; one or more biomathematical models, i.e. ZeitZeiger model, LASSO model PLS, RNA expression data comprising data points relating to the sample provided by the subjects and/or the subject such that an update uses the data; one or more algorithms using one or more of the parameters, one or more biomathematical models, i.e. ZeitZeiger model, LASSO model PLS, and the data as input, such that the algorithms enable determination of the circadian rhythm, amplitude, circadian phase; and code to implement the algorithms.

Also provided are one or more processors in communication with the processor-readable medium and configured to execute the code. In some embodiments of the present invention, a method is provided for applying a biomathematical model that relates a state to time and/or time of sampling of a subject.

In some embodiments of the present invention, there is provided a method for applying a biomathematical model that relates a condition of a subject providing the sample to circadian rhythm disorder.

In some embodiments of the present invention, the mathematical model uses the R program. In some embodiments of the present invention, there is provided a method for implementing a biomathematical model that produces and updates a mathematical relationship between a RNA expression data set of a sample, parameters related to subject, amplitude value, circadian phase value, score, weight factor, and a time, i.e. time of sampling.

In some embodiments of the present invention, there is provided a method for implementing a biomathematical model that produces and updates a mathematical relationship between a RNA expression data set of a sample, parameters related to subject, amplitude value, circadian phase value, score, weight factor, and a circadian rhythm disturbance or circadian rhythm related disorder.

In embodiments, the procedure includes: 1) selecting a sample type of a subject 2) selecting the RNA expression level of circadian phase genes and amplitude prediction genes obtained from selected sample and parameter of the subject; 3) selecting at least one biomathematical model, preferably a ZeitZeiger, LASSO, partial least square; 4) selecting at least one parameter related to the biomathematical model; 5) selecting a training data set; 6) loading training data set data entries of reference gene expression levels (from other subjects), comprising gene expression levels from circadian phase genes and amplitude prediction genes, wherein each data entry comprises a gene label, a weight factor, and an amplitude score and/or time parameters; 7) inputting the RNA expression levels of circadian phase genes and amplitude prediction gene from a sample and subject's parameters (i.e. time of sampling, age, gender; 8) calculating a circadian phase value for the subject by applying the weight factor of each circadian phase gene from the data entries in step 6) to each circadian phase gene expression level inputted in step 7; 9) calculating an amplitude value for the subject by applying the weight factor of amplitude prediction genes from the data entries in step 6) to each amplitude prediction gene expression level inputted in step 7; 10) computing the phase of the circadian rhythm by applying a time of sampling for the sample provided by the subject to the phase value from step 8 and matching the phase value to the time parameters of the training data set from step 6); 11) computing the amplitude of the circadian rhythm by matching the amplitude value from step 8) with the amplitude score of the training data set from step 6; 12) optional: comparing results to a control having a non-disturbed circadian rhythm and/or a non-diseased control; 13) determining a subject's circadian rhythm by applying circadian phase from step 10 and amplitude from step 11 and 12; and 14) repeating steps 1,2, 7 through 12 as needed to update the biomathematical model during determination of a subject's circadian rhythm, for example with different biomathematical models, an updated training data set, amplitude score, and/or weight factor.

In some embodiments of the present invention, there is provided a processor-readable medium comprising code representing instructions for causing a processor to use in one or more biomathematical model executing steps 1-14 as described above.

The term "input" as used herein, describes a function to enter data for applying a mathematical model and returns a reference to the data in the form of a string. Data comprise for example RNA quantities from circadian phase genes, relative RNA expression levels from circadian phase genes, RNA quantities from amplitude prediction genes, relative RNA expression levels from amplitude prediction, gene labels, individual parameter labels, numeric individual parameter, clinical laboratory parameter labels, numeric clinical laboratory parameter, amplitude score, weight factor. The term "output", as used herein, describes a value produced by an algorithm, preferably a human readable value, more preferably a value defining a circadian rhythm, an amplitude, a circadian phase.

The term "plurality", as used herein, means the state of being numerous.

Through a mathematical model, problems and its instances (in the algorithmic sense) may be formulated and analyzed for properties. An algorithm is usually a step-by-step process with well-defined steps and takes an input instance of a problem instance (a mathematical model) and produces an output, wherein an algorithm can be implemented into a computer program. A computer program is generally a series of instructions, complying with the rules of a particular programming language, to perform or solve certain functions or tasks or problems with the help of a computer.

The term "execute" also means the process by which a computer or virtual machine executes the instructions of a computer program, written in a programming language, to see the output, including, wherein the programming language can be selected from a group of R, Java, Fortran, C, C++, Python, C#, JavaScript, VB .NET, PHP, High Level Shading Language, and MATLAB. In one embodiment, the mathematical modelling algorithm for calculating circadian rhythm, amplitude, circadian phase is programmed and executed in R or any distribute of R.

### Training data set, reference data

The term "reference data" as used here includes primary data for all inputs, parameters, absolute RNA quantities, relative RNA quantities, gene labels, scores, weight factors, model variables (dependent or independent), even under different sampling times and subject condition. In some embodiments, reference data comprise published experimental data of human samples comprising literature data, experimental data of human sample at optimal circadian rhythm and/or experimental data of human sample at disturbed circadian rhythm with or without a circadian rhythm related disorder. Reference data sets are usually stored in databases.

In one embodiment, calculating the amplitude and the phase of the circadian rhythm is performed using a biomathematical model based on the machine-learning ZeitZeiger algorithm. In one embodiment, calculating the amplitude and the phase of the circadian rhythm is performed using a biomathematical model based on the machine-learning ZeitZeiger algorithm.

### FIGURES

The invention is demonstrated by way of the example through the figures disclosed herein. The figures provided represent particular, non-limiting embodiments and are not intended to limit the scope of the invention.

Short description of the figures:
**Figure 1****: Study strategy for developing a hair root cell based assay to predict DLMO**
**Figure 2****: Pilot study: Gene expression rhythms in hair roots from three healthy controls**
**Figure 3****: HairTime study: Gene expression rhythms in hair roots from 13 healthy controls**
**Figure 4****: Significant correlation between predicted DLMO from hair root cells with DLMO measured in saliva samples.**
**Figure 5****: Significantly rhythmic genes over the 24 hour day using MetaCycle analysis (HairTime study).**
**Figure 6****: Amplitude estimation using gene expression levels obtained in Hairtime Study.**
**Figure 7****: Amplitude prediction**
**Figure 8****: Amplitude correction**
**Figure 9****: ZeitZeiger model resulted in a different number of features and corresponding loadings**
**Figure 10****: LASSO models**
**Figure 11****: Amplitude prediction for low amplitude samples**
**Figure 12****: New LASSO models specifically designed for low-amplitude samples**

Detailed description of the figures:

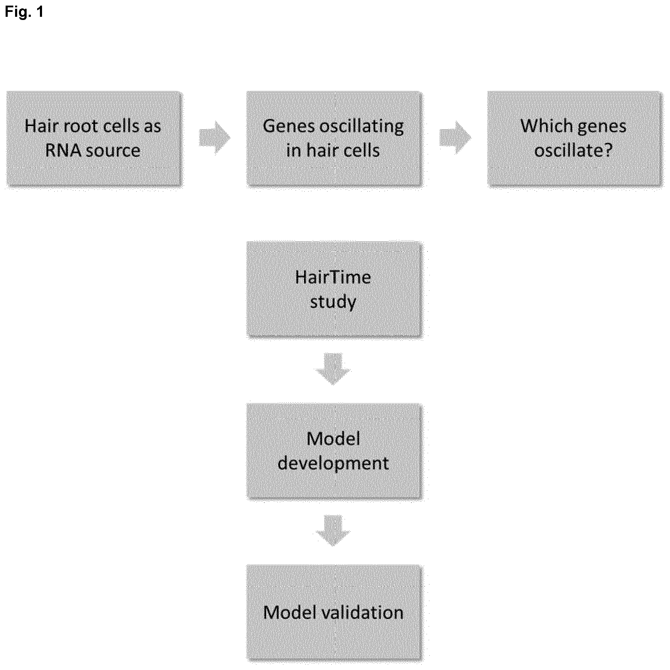

**Figure 1****: Study strategy for developing a hair root cell based assay to predict DLMO**

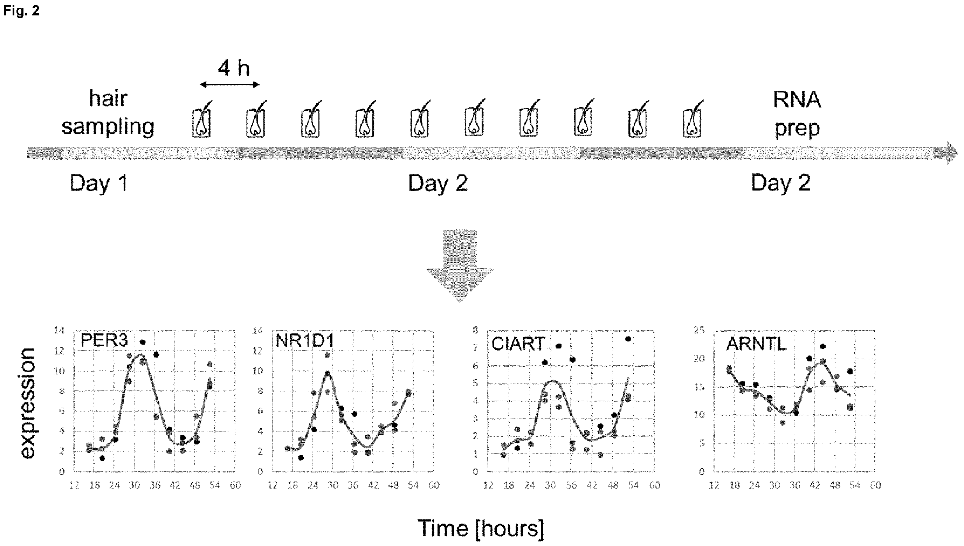

**Figure 2****: Pilot study: Gene expression rhythms in hair roots from three healthy volunteers.** Gene expression analysis of selected genes in human hair root cells. RNA from hair roots taken in regular 4-hour from three intervals (different colors) was analyzed for gene expression using NanoString analysis. Note, selected rhythms show (i) similar phase, (ii) similar amplitude, and (iii) similar expression level - important features for phase-predicting biomarker genes. Gene expression was normalized to the expression of CLTC (a housekeeper gene).

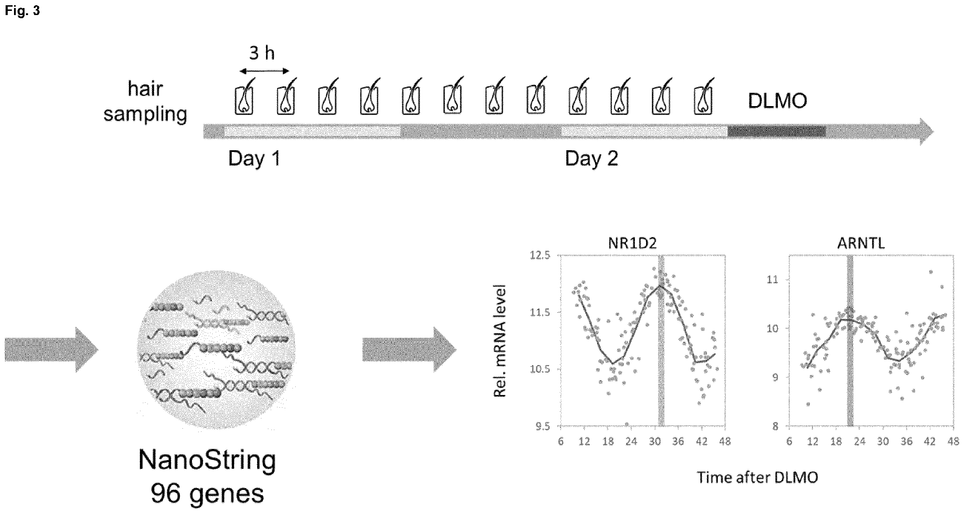

**Figure 3****: HairTime study: Gene expression rhythms in hair roots from 13 healthy controls.** Genes, robustly rhythmic with a stable phase (relative to DLMO) among several individuals were identified in hair roots samples from 13 healthy subjects (male and female; aged 22-52years) taken in regular 3 hour intervals over 36 hours. Transcript levels from isolated RNA were detected using RNA-Seq. The expression of 96 from RNAseq preselected genes was analysed using NanoString. DLMO was determined using saliva samples collected at the same evening as hair sampling from each subject. DLMO was used in further analysis. Each gene expression value was assigned to a time stamp relative to DLMO.

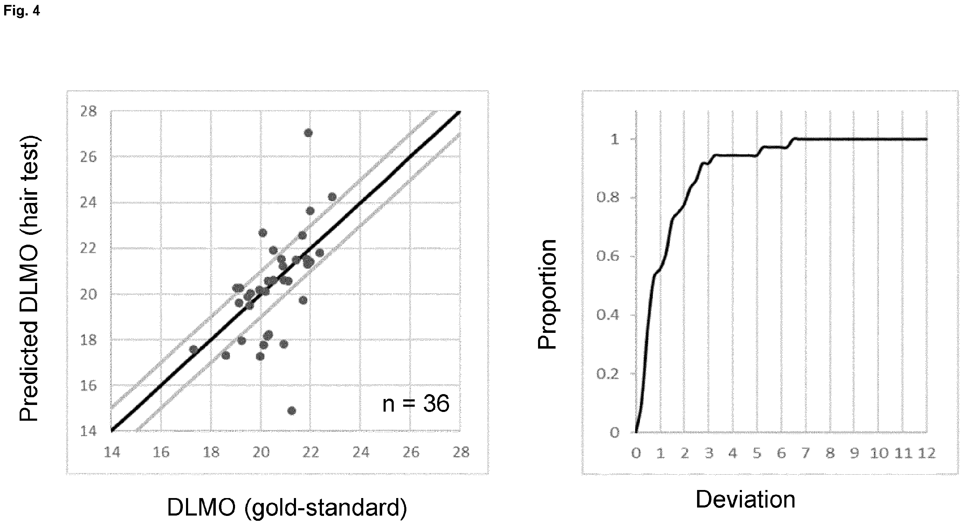

**Figure 4****: Significant correlation between predicted DLMO from hair root cells with DLMO measured in saliva samples.** Right: ROC curve showing that 50% of the samples are predicted with a deviation less than 0.75 hours, 75% of the samples with a deviation less than 2 hours etc.

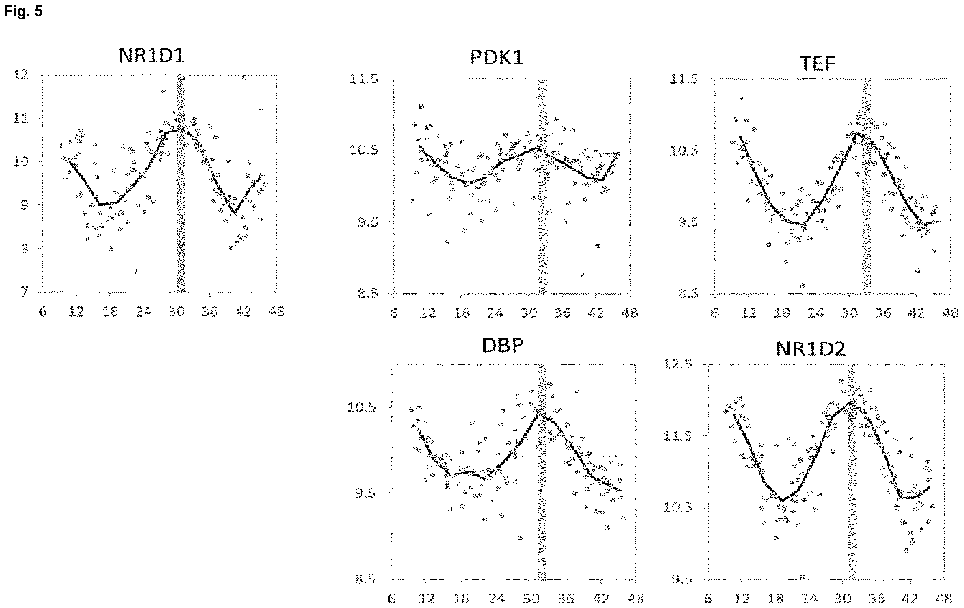

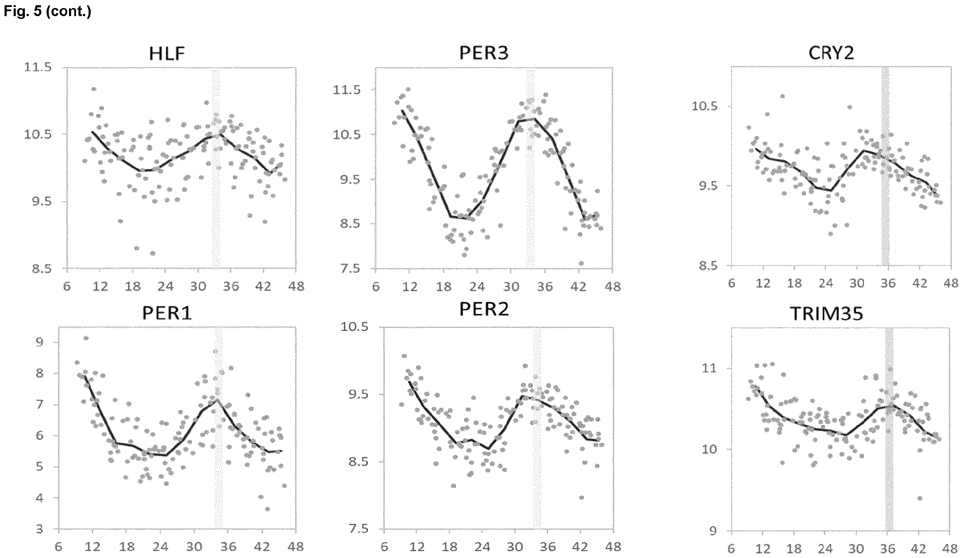

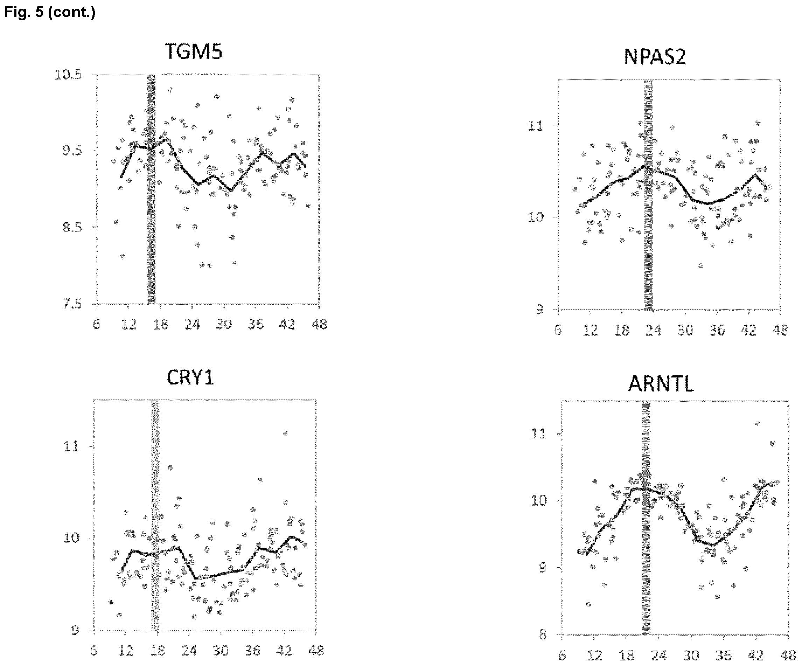

**Figure 5****: Significantly rhythmic genes over the 24-hour day using MetaCycle analysis (HairTime study).** () From 96 differently expressed genes, the circadian phase prediction genes were selected to predict the time stamp of each sample. Three different published algorithms (ZeitZeiger, Partial Least Square, LASSO), also termed BodyTime algorithm, were trained with data from the HairTime study to identify gene sets and parameters that best predict the individual samples. The leave-one-out cross validation was used to estimate the performance of each algorithm for prediction. For each oscillatory circadian phase gene, the peak of expression corresponds to specific times over the 24-hour day. 16 genes are significantly rhythmic when averaging over all individuals (MetaCycle analysis).

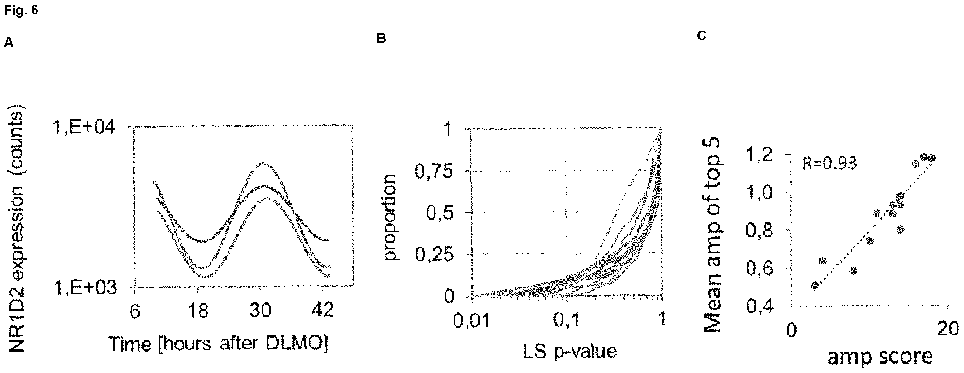

**Figure 6****: Amplitude estimation using gene expression levels obtained from a single sample.** Amplitude of a rhythm was estimated using RNA expression levels of 90 genes from a single sample (instead of measuring a time series) obtained from 13 healthy subjects. The amplitude was defined for example as: (i) number of genes significantly rhythmic with a given p-valus; (ii) number of genes with a minimal amplitude; (iii) mean amplitude of the top5 or top10 rhythmic genes.

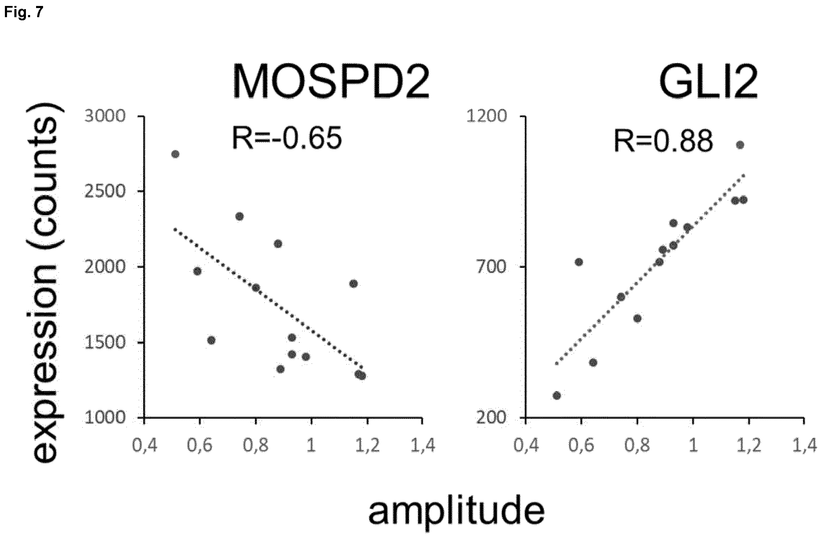

**Figure 7****: Amplitude prediction.** Using LASSO, genes and corresponding parameters were identified whose expression correlate with amplitude and can be used to predict an amplitude. Amplitude prediction genes are not rhythmic by themselves but show a correlation of their expression with amplitude of the subject.

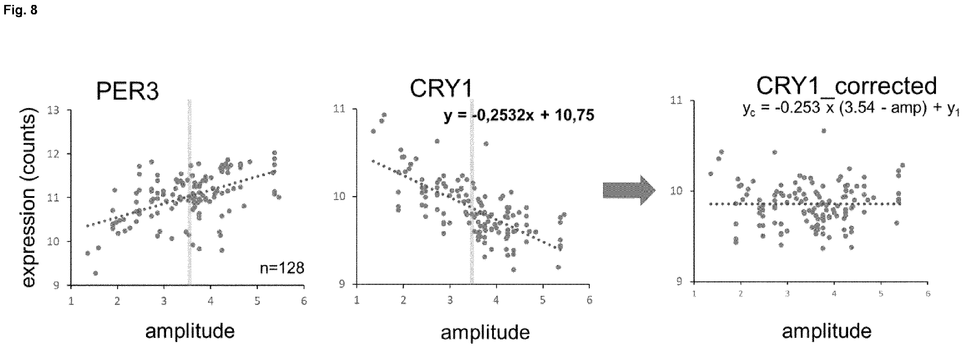

**Figure 8** **Amplitude correction.** Beside amplitude prediction genes, other genes including the predictor genes for circadian phase, correlate with amplitude, which may harm a correct circadian phase prediction. Therefore, an amplitude correction was performed according to amplitude level (only for subjects with amplitudes <2) using correction data (slopes).

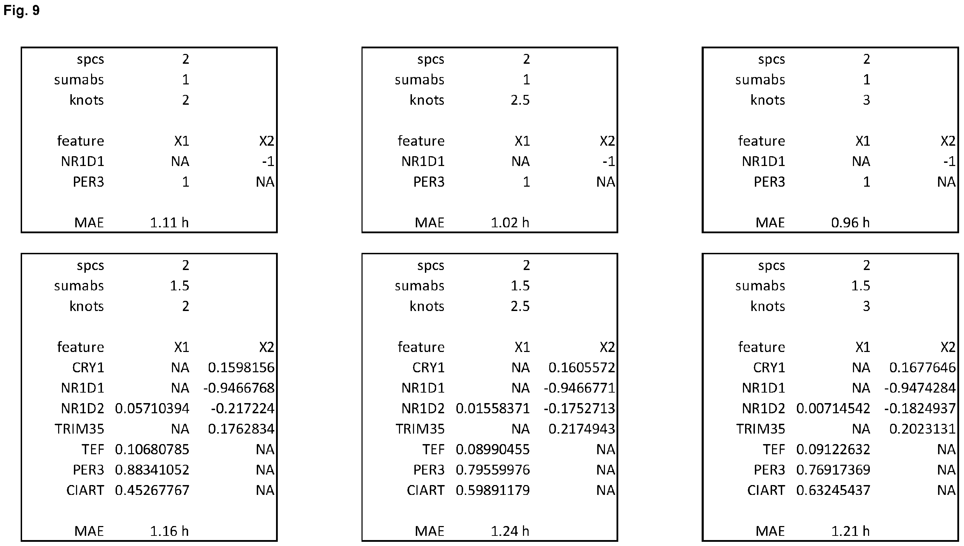

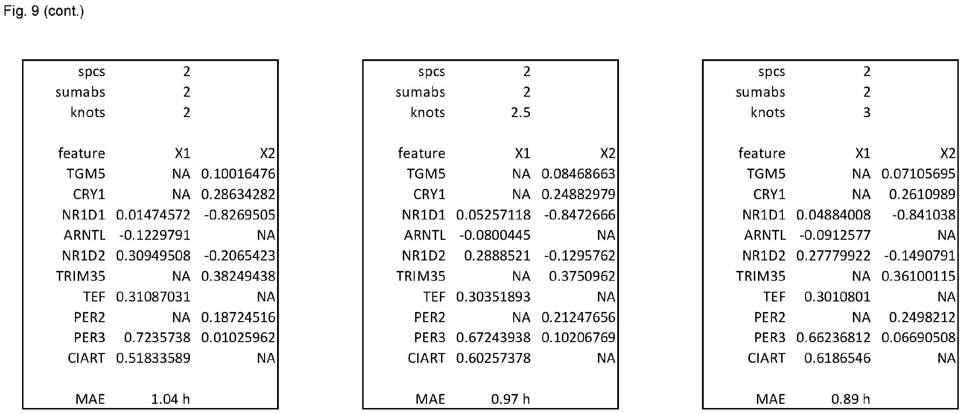

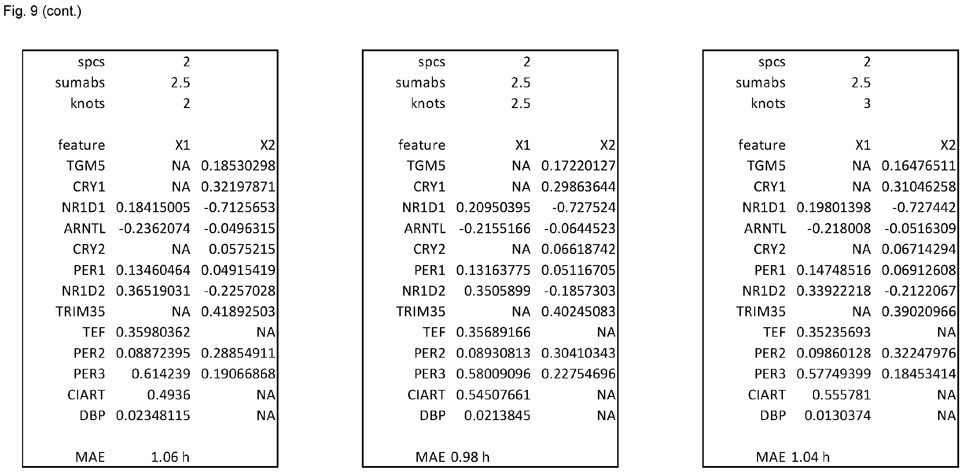

**Figure 9****: ZeitZeiger model resulted in a different number of features and corresponding loadings.** 12 ZeitZeiger models were trained using data from HairTime study with various indicated parameter combinations (spc, sumabs, knots). Identified features and loadings for the two sparse principal components (X1, X2) are given. MAE: median absolute deviation of model-predicted DLMO from measured DLMO for the 36 samples from the validation SleepShift study.

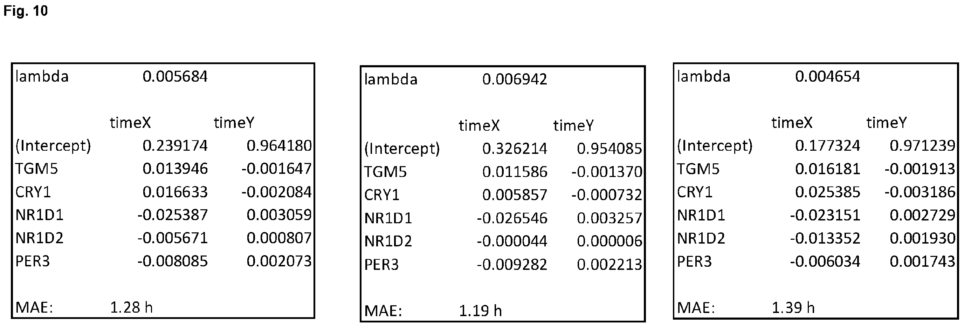

**Figure 10****: LASSO models.** Three LASSO models were trained using data from HairTime study with indicated parameter lambda. Identified features and loadings for the two time components are given. MAE: median absolute deviation of model-predicted DLMO from measured DLMO for the 36 samples from the validation SleepShift study.

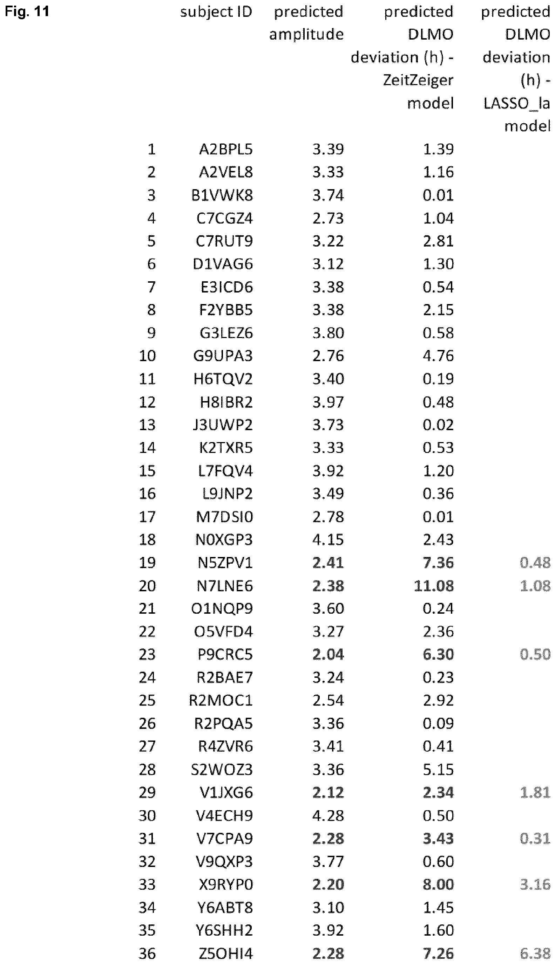

**Figure 11****: Amplitude prediction for low amplitude samples.** For the 36 samples from the SleepShift study, amplitudes were predicted using the developed LASSO model. Samples with predicted amplitudes smaller than 2.5 are marked in red. They display also low accuracy when DLMO is predicted using the ZeitZeiger models. Prediction gets much better with new LASSO models specifically designed for low-amplitude samples.

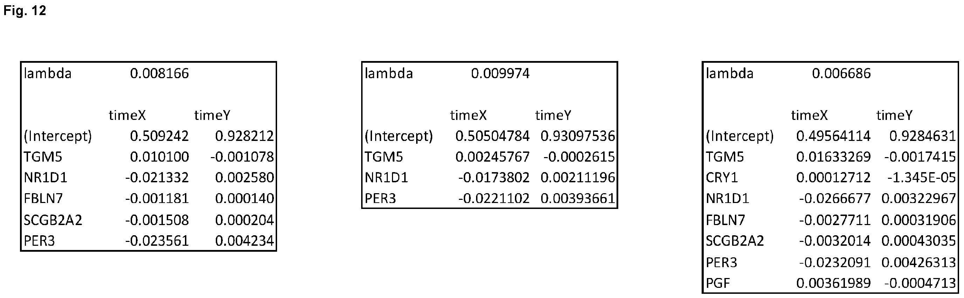

**Figure 12****: New LASSO models specifically designed for low-amplitude samples.** Identified features and loadings for the two time components are given. Predicted DLMO deviation for identified low-amplitude samples are given in Figure 11.

### EXAMPLES

The invention is demonstrated through the examples disclosed herein. The examples provided represent particular embodiments and are not intended to limit the scope of the invention. The examples are to be considered as providing a non-limiting illustration and technical support for carrying out the invention.

Described in more detail below is:
- Methods, study design and characteristics of patient cohorts analysed
- A pilot experiment in which RNA from hair root samples are sequenced to identify and select rhythmicity of transcripts
- HairTime study for (i) predicting circadian phase genes and (ii) developing trained biomathemathical prediction models that predicts DLMO
- SleepShift study: for evaluating performance of circadian phase predictors and detection and prediction of amplitude predicting genes
- Validation study in controls and insomnia patients
- Description of the procedure for determining the circadian rhythm from a subject providing a sample
- Prevention, diagnostic, and treatment of circadian rhythm disturbance

### Example 1: Development of a hair root cell based assay to predict DLMO

The inventors have developed a hair test through an extensive study. The plan of the study is shown in Figure 1. The most critical step of this study was to identify a set of genes within the more than 20,000 genes in our body that are capable of detecting the exact individual body clock phase as well as the amplitude through a single sample. In the validation studies, we were able to demonstrate that our algorithm, which combines the gene activity values of the identified group of genes, has similar accuracy in predicting an individual's chronotype as the current gold standard method, namely the burdensome dim light melatonin onset (DLMO) test.
1. Determine rhythmic transcripts in human hair root samples.
   In pilot experiments, three time series have been collected (10 samples each every 4 hours). RNA was prepared and sequenced and rhythmicity of transcripts was analyzed using MetaCycle (PMID: 27378304). (Figure 2)
2. Select rhythmic candidate genes for a focused analysis
   90 rhythmic candidate genes (and 6 housekeeper genes) for NanoString analysis were selected based on specific criteria (e.g. minimal expression level: 1.5; minimum amplitude 0.1, among top 500 in p-value rank, etc.). This is the list of 90 candidate genes:
   GOT1, NDUFAF4, CDPF1, SPRY2, ZBTB42, HLF, ARSJ, RBM18, TGM5, GNB1L, CRY1, ANO7L1, GL12, NR1D1, ZNF669, FBLN7, CD34, NME7, RORC, TNS1, STXBP4, LRRC37A3, BORCS6, ZSCAN31, SLC6A6, NR1H3, PIF1, ARNTL, POLR3GL, DUBR, KRT15, NPAS2, PSMG3-AS1, AC011476.3, HERC3, MOSPD2, SPRR2B, ZNF510, SLC22A15, VWF, AL391121.1, CRY2, MAP3K14, POLR2J4, PER1, CLEC11A, ZNF296, SERPINE2, PDE6B, PLEKHF1, PDSS1, B3GNT2, MINOS1, RAI14, TDRKH, P4HA1, SAP30, KLRG2, AC016727.1, C1orf123, CDCA3, NR1D2, TRIM35, TEF, DI030S, LRAT, RRAD, AC048341.2, POLR2I, SCGB2A2, LAYN, ZNF749, CAMKMT, CNN1, PER2, C12orf66, PER3, URB1-AS1, CIART, CDC25B, SPRR2A, RPL23AP7, PIBF1, U2AF1, PDK1, DBP, PGF, PRR34-AS1, ALDH3A1, TLR5

### These are the 6 housekeeper genes: PGK1, CLTC, PSMB2, PPIA, HPRT1, GAPDH

NanoString analysis with this 96-panel was performed on the three test time series to evaluate whether rhythmicity of selected genes is similar to that detected using RNA-Seq.

### 3. HairTime study I: collect and process samples

To identify biomarkers predicting internal phase, the HairTime study was performed in 13 individuals (male and female; age: 22-52y) provided hair cells every three hours, 12 samples each. RNA was extracted and NanoString analysis performed using the panel described above. DLMO was determined using saliva samples collected at the same evening as hair sampling Saliva was analysed using a Bühlmann ELISA. For analysis, Hockey-stick_v2.4 was used and Hockey-stick DLMO was used for further analysis. (Figure 3)

### 4. HairTime study II: select features that predict DLMO and develop prediction models

ZeitZeiger (PMID: 26819407) models were implemented using LOG2 normalized expression data and timestamps (hours after DLMO) generated using sampling time and DLMO. (Figure 4)

All samples (156 - 10 with quality issues = 146) were selected for training with leave-one-out cross validation (CV). The following genes were not considered, either because their corresponding NanoString probes had manufacturing quality issues or they were lowly expressed in most samples:
AC011476.3, AL391121.1, ANO7L1, CD34, DlO3OS, PIF1, PLEKHF1, POLR2J4, POLR3GL, PRR34-AS1, PSMG3-AS1, RORC, PGK1, PIBF1, POLR2I, RAI14, RBM18, MINOS1.

Either LOPO or LOSO (leave-one-proband/sample-out) cross-validation was performed using the ZeitZeiger routine to determine the best ZeitZeiger parameters for subsequent model generation. Best parameters were sumabs: 2.5; Spc: 2 and knots 2 or 3. However, other parameters also gave good results (median absolute prediction error (MAE): 1.3-1.7 h). We sought to assess whether selecting a particular parameter set would lead to overfitting.

Thus, 12 ZeitZeiger models were trained for subsequent prediction with only Spc = 2 being fixed and sumabs not exceeding 3 to keep number of genes low. Selected parameters: sumabs: 1, 1.5, 2, 2.5. Knots: 2, 2.5, 3.

LASSO models (PMID: 30201705) were also implemented using this data set by and performed a little worse in LOSO cross validation compared to ZeitZeiger model. Again, to have more than one LASSO model, the CV-determined lambda value was varied to create three LASSO models (In(lambda); In(lambda)+0.2; In(lambda)-0.2). Those models have subsequently been tested using validation data derived from an independent study (SleepShift).

### 4. SleepShift study: evaluate performance of predictors

The SleepShift study recruited 41 healthy male and female control aged 23 to 65. For three subjects, no RNA could be retrieved. For two, no DLMO could be determined. 36 individuals have been analyzed regarding DLMO (from saliva) and regarding gene expression from hair root cells. Performance of the ZeitZeiger and LASSO models were tested using LOG2 normalized data (NanoString) and timestamps (hours after DLMO).

### 5. ZeitZeiger and LASSO models

ZeitZeiger models: As described above, for ZeitZeiger prediction, 12 models were trained for subsequent prediction with only Spc = 2 being fixed and sumabs not exceeding 3 to keep number of genes low. Selected parameters: sumabs: 1, 1.5, 2, 2.5. Knots: 2, 2.5, 3. Each ZeitZeiger model resulted in a different number of features and corresponding loadings (Figure 9).

Averaging the predicted DLMO values for all 12 models resulted in an MAE (median absolute deviation) of 1.25 hours. We noticed that the prediction accuracy was especially low for seven (of 36 samples). For these 7 samples MAE was 7.26 hours. Without these seven samples, prediction accuracy of the remaining 29 samples was MAE = 0.6 hours. Thus, these seven samples have been looked at specifically and a separate model was determined for these types of samples. Accordingly, three LASSO models were developed (Figure 10).

LASSO models: As describe above, to have more than one LASSO model, the CV-determined lambda value was varied to create three LASSO models (In(lambda); In(lambda)+0.2; In(lambda)-0.2. Those models have been tested using validation data.

The seven samples with low prediction accuracy in ZeitZeiger were predicted much better with the LASSO models (MAE: 2.4 hours).

Thus, LASSO models seem are better suited to predict such kind of samples. We suspected that they were derived from subjects with low circadian amplitudes.

### 6. Detection and prediction of amplitude differences

Selected genes from the 13 time-series of the HairTime study (CRY1, NR1D1, ARNTL, NPAS2, CRY2, PER1, NR1D2, TEF, PER2, PER3, CIART, DBP) were analyzed for rhythmicity using sine fits. Amplitude of five most robustly rhythmic genes (NR1D1, NR1D2, TEF, PER3, CIART) served as indicator of oscillator amplitude in hair root cells. (Figure 5, Table 1)

Amplitude of a rhythm was estimated using RNA expression levels of 90 genes from a single sample (instead of measuring a time series) obtained from 13 healthy subjects. The amplitude was defined for example as: (i) number of genes significantly rhythmic with a given p-values; (ii) number of genes with a minimal amplitude; (iii) mean amplitude of the top5 or top10 rhythmic genes. (Figure 6).

LASSO was performed to extract predictor genes for amplitude. Only morning timepoints from HairTime samples (ranging from 6am to noon for an average sampling begin of 9am in our study) were used for training, i.e. 64 samples. Only non-low expressing genes, genes without quality issues in their probes (see above) and genes with low variation in their expression over time (to avoid rhythmic and "noisy" genes [i.e. geomean of relative standard deviation < 25%] were included. These were 46 genes (AC016727.1, AC048341.2, B3GNT2 , BORCS6 , C12orf66 , C1orf123 , CAMKMT , CDC25B , CDPF1 , CLEC11A, CRY1 , CRY2 , DUBR , FBLN7 , GLI2 , GNB1L , GOT1 , HERC3 , HLF , LAYN , LRRC37A3 , MAP3K14 , MOSPD2 , NDUFAF4 , NME7 , NPAS2 , NR1H3 , P4HA1 , PDE6B , PDK1 , PDSS1 , SAP30 , SLC22A15 , SPRY2 , STXBP4 , TLR5 , TRIM35 , U2AF1 , URB1.AS1 , VWF , ZBTB42 , ZNF296 , ZNF510 , ZNF669 , ZNF749 , ZSCAN31)

**Table 1: Amplitudes of gene expression rhythms (from HairTime time series) analyzed with fitting a sine wave to LOG2 data. Gene transcripts indicated in yellow were most robustly rhythmic and correlated best to each other. Thus, these amplitudes were calculated in a subject's oscillator amplitude ("five genes").**

| **subject ID** | **CRY1** | **NR1D1** | **ARNTL** | **NPAS2** | **CRY2** | **PER1** | **NR1D2** | **TEF** | **PER2** | **PER3** | **CIART** | **DBP** | **five genes** |
|---|---|---|---|---|---|---|---|---|---|---|---|---|---|
| **R3** | 1.241 | 2.951 | 1.522 | 1.071 | 1.504 | 2.437 | 2.048 | 1.757 | 1.910 | 3.405 | 2.547 | 1.329 | 2.471 |
| **07** | 1.945 | 4.952 | 3.153 | 2.193 | 1.215 | 5.916 | 3.122 | 3.705 | 2.472 | 8.246 | 4.577 | 1.998 | 4.645 |
| **L3** | 1.784 | 1.073 | 1.852 | 1.300 | 1.346 | 5.864 | 1.720 | 2.515 | 1.944 | 4.065 | 1.308 | 1.844 | 1.899 |
| **18** | 1.147 | 4.429 | 1.665 | 1.325 | 1.360 | 5.140 | 3.130 | 2.258 | 1.637 | 7.052 | 3.372 | 1.661 | 3.753 |
| **J6** | 1.279 | 3.097 | 1.912 | 1.125 | 1.929 | 16.602 | 3.090 | 3.050 | 2.043 | 8.951 | 2.495 | 2.196 | 3.654 |
| **J7** | 1.616 | 7.020 | 1.900 | 1.378 | 1.864 | 4.085 | 4.461 | 3.706 | 2.075 | 9.097 | 4.236 | 2.380 | 5.372 |
| **Z7** | 1.015 | 3.172 | 1.769 | 1.303 | 1.113 | 2.943 | 2.424 | 1.783 | 1.398 | 5.463 | 2.029 | 1.444 | 2.731 |
| **S4** | 1.298 | 5.600 | 1.692 | 1.383 | 1.416 | 3.116 | 3.793 | 2.701 | 1.689 | 5.966 | 2.618 | 1.927 | 3.895 |
| **01** | 1.421 | 3.361 | 2.265 | 1.456 | 1.153 | 3.011 | 2.481 | 1.972 | 2.014 | 5.417 | 2.151 | 1.382 | 2.861 |
| **M7** | 1.881 | 3.991 | 4.007 | 1.393 | 1.610 | 8.095 | 3.919 | 3.224 | 1.986 | 9.560 | 3.302 | 1.694 | 4.369 |
| **Q3** | 1.154 | 4.483 | 1.367 | 1.221 | 1.889 | 3.827 | 3.056 | 2.318 | 1.897 | 5.186 | 3.743 | 1.873 | 3.614 |
| **Y7** | 1.438 | 2.145 | 1.327 | 1.081 | 1.457 | 3.651 | 2.199 | 2.184 | 1.507 | 3.518 | 2.207 | 1.879 | 2.402 |
| **G8** | 1.203 | 6.244 | 2.240 | 1.574 | 1.453 | 5.377 | 3.325 | 2.530 | 1.684 | 6.501 | 4.020 | 1.843 | 4.242 |

Other amplitude prediction models are likely to exist. For lambda=0.15 (trade-of between CV accuracy and number of genes), 6 predictor genes were selected (DUBR, GLI2, MOSPD2, PDK1, ZNF296, ZNF749).

Amplitudes for the 36 validation samples from the SleepShift were predicted using the genes and loading from the LASSO model given Table 2.

**Table 2: The LASSO model was trained using amplitude data from HairTime. Identified features and loadings are given.**

| **(Intercept)** | **-0.3400000** |
|---|---|
| CRY1 | 0.0008000 |
| CRY2 | 0.0003700 |
| DUBR | -0.0007000 |
| GLI2 | 0.0042000 |
| MOSPD2 | -0.0007500 |
| PDK1 | 0.0017000 |
| SPRY2 | 0.0002000 |
| TLR5 | 0.0006000 |
| TRIM35 | 0.0001300 |
| ZNF296 | 0.0010000 |
| ZNF749 | -0.0006500 |

Amplitudes lower than 2.5 were classified as "low amplitude". These was the case for seven samples - exactly those with low prediction accuracy in the ZeitZeiger models (Figure 11).

As shown in Figure 7, genes and corresponding parameters were identified using LASSO whose expression correlate with amplitude and can be used to predict an amplitude. Amplitude prediction genes are not rhythmic by themselves but show a correlation of their expression with amplitude of the subject.

Beside amplitude prediction genes, other genes including the predictor genes for circadian phase, correlate with amplitude, which may harm a correct circadian phase prediction. (Figure 8) Therefore, an amplitude correction (Table 3) was performed according to amplitude level (only for subjects with amplitudes <2) using correction data (slopes). Thus, amplitudes lower than 2.5 were classified as low amplitude. These was the case for seven samples - exactly those with low prediction accuracy in the ZeitZeiger models.

**Table 3: Slopes**

| **Gene** | **Slope** |
|---|---|
| SPRY2 | 0.1570 |
| TGM5 | -0.0810 |
| CRY1 | -0.1727 |
| GLI2 | 0.4311 |
| NR1D1 | -0.1142 |
| ARNTL | -0.1899 |
| DUBR | 0.0791 |
| KRT15 | 0.3217 |
| MOSPD2 | -0.2378 |
| CRY2 | 0.0181 |
| PER1 | 0.2523 |
| ZNF296 | -0.0447 |
| NR1D2 | 0.0571 |
| TRIM35 | 0.0525 |
| TEF | 0.1936 |
| ZNF749 | -0.0760 |
| PER2 | 0.0984 |
| PER3 | 0.2288 |
| CIART | 0.1065 |
| PDK1 | 0.1992 |
| DBP | 0.0552 |
| PGF | -0.3580 |
| TLR5 | -0.0015 |

### 7. Conclusion and final models

Predicted DLMO for all 3 models were averaged and median deviation from DLMO was calculated. (Figure 12)

Since low-amplitude samples can be identified using LASSO amplitude prediction model, model usage depends on prior amplitude prediction.

Combined accuracy for all 36 samples (using ZeitZeiger models for high-amplitude and LASSO_la models for low-amplitude samples) is: MAE: 0.82 hours. There is a highly significant correlation (Figure 4).

### Example 2: Procedure for determining the circadian rhythm from a subject providing a sample

The procedure for determining the circadian rhythm from a subject providing a sample is also termed "BODYTIME". BODYTIME is an innovative and differentiated test, such as hair test, to help people find out their individual body clock (chronotype) accurately and in a simple way:
Receiving sample of the subject: The subject ordered a home test kit through the website. The test kit comprises a sample receiving tool, a sample receiving tube containing a sample buffer solution (if applicable), instruction sheet for use, safety data sheet (if applicable), a label, and an addressed return envelope. Once the test kit has arrived at the subject's home, he/she withdraws a hair root sample or saliva sample or oral mucosa sample, transfers the sample into the RNA Later-like Buffer Solution, if applicable, and sends the sample back to the laboratory.

RNA isolation from sample and sequencing: In the laboratory, we measure the gene activity / RNA expression levels of circadian phase genes and amplitude prediction genes (as investigated in Example 1) in the hair root sample or oral mucosa sample or salivary sample. Total RNA is isolated from each sample (hair root, oral mucosa, salivary) using the TRIzol reagent (Thermo Fisher) according to the manufacturer's instructions. All samples were quantitated and quality controlled with the NanoDrop 2000 Spectrophotometer (Thermo Scientific) and the Qubit 4.0 Fluormeter (Thermo Scientific). Before 3'-end RNA-sequencing, all samples were DNAse-treated. For 3'- end RNA-sequencing, the inventors used the protocol described in Shishkin et al. (Shishkin et al., Nat Methods, 2015 Apr; 12(4):323-5) with the exception of polyN selecting (using Dynabeads^{™} Oligo(dTh5, Thermo Fisher, according to the manufacturer's instructions) after pooling the samples into a single tube, instead of rRNA depletion step.

Gene expression analysis using NanoString. The isolated RNA was subjected to NanoString nCounter^{™} Gene Expression analysis (NanoString Technologies, Seattle, WA, USA) using nCounter^{®} Elements^{™} TagSets, according to the manufacturer's protocol. Probes for the circadian phase genes, the amplitude predicition genes and the housekeeping genes GAPDH, H PRT1, PSMB2 and PPIA were designed and by NanoString Technologies and synthesized by Integrated DNA Technologies (Leuven, Belgium). Raw data were analysed using nSolver^{™} software (NanoString Technologies) using standard settings and was normalized against the housekeeping genes or against the median RNA expression level of all RNA transcripts detected.

Data analysis. The sequenced data was aligned to the human genome (hg19) using STAR short-sequence aligner (Dobin et al. 2013, Bioinformatics, 2013 Jan 1; 29(1 ):15-21) with standard settings such that only the uniquely mapped reads were retained. These uniquely mapped reads were assigned to RefSeq genes using the ESAT tool that was designed for quantification of 3'-end RNA-Seq (Derr et al., Genome Res, 2016 Oct; 26(10):1397-1410). Only libraries with at least 2 million total mapped and assigned 3'-end reads were used for further analysis. Libraries were normalized using the total number of mapped reads and corrected for weighted trimmed mean of M-values (Robinson and Oshlack, 2010, Genome Biology 11, R25). The resulting count data for each library was analyzed for circadian genes by linear regression of sinusoids using edgeR (Robinson et al., Bioinformatics, 2010 Jan 1; 26(1):139-40). For supervised sparse PCA analysis, the count data was converted to normalized counts-per-million data using edgeR.

The algorithm then calculates the circadian phase and amplitude for determining the circadian rhythm/ body clock (chronotype) of the subject. To identify circadian phase genes and amplitude prediction genes (Example 1) we applied the LASSO method of Hughey et al. (2016). Central to the ZeitZeiger method is a supervised sparse PCA that reduces the variation associated with the periodic variable in the training set (in our case: internal time) to a low-dimensional subspace (two sparse principal components: SPC1, SPC2) of the hair root transcriptome. The LASSO and PLS algorithm were also applied. RNA expression levels of the subject for the circadian phase genes and amplitude prediction genes identified, were applied to the biomathematical model ZeitZeiger and/or LASSO and/or PLS being trained with reference data set as obtained in Example 1. Using
The body clock (chronotype) of the subject plus a personalized schedule with timing recommendations for sleeping, working, eating, exercising, etc. is send to the subject through the web application.

The most critical element in this process was the identification of a set of genes within the more than 20.000 genes in our body, which have the ability to reveal the exact individual body clock. During the validation studies, we could prove that our algorithm combining the gene activity levels of the identified set of genes shows a similarly high accuracy in predicting a subject's circadian rhythm (chronotype) to today's gold-standard-method, namely the burdensome Dim-Light-Melatonin-Onset (DLMO) test.

### Example 3: Validation study in controls and insomnia patients

A validation study was performed to test the accuracy of the circadian phase prediction in an independent cohort.

Sample: A hair root was sampled from 48 healthy controls and 27 insomnia patients (=75 subjects in total) at one time point. The RNA extraction, RNA quality control, and NanoString method for determining RNA expression levels in circadian phase genes and amplitude prediction genes were performed as described in Example 2. The DLMO was determined for each sample. RNA expression levels from each subject was applied to BodyTime algorithms (ZeitZeiger, LASSO, PLS) for calculating circadian phase and amplitude to determine the circadian rhythm for each subject.

Performance of BodyTime algorithms is slightly different, depending on which subcohort is analysed, but achieved accuracy of 0.7-1 hour (median deviation from gold-standard phase marker, DLMO) (Figure 8b). For subjects with low amplitude, performance substantially improves after amplitude correction as described above.

### Example 4: Prevention, diagnostic, and treatment of circadian rhythm disturbance

### Prevention:

More and more people proactively seek ways to optimize their health and prevent diseases. While sleep will be our first target market in the prevention space, it represents only a fraction of the market opportunities available for our technology. Our innovative solution around chronobiology can function as a new lever for many other preventative fields where timing plays an important role:
- Intermittent fasting: ~13 million people in Germany already tried it
- Sports performance: ~11 million people in Germany exercising regularly and trying to improve their performance
- General self-optimization: ~18 million people (18-65 years) in Germany use apps and / or tracking devices to live healthier (Thompson, Walter R. Ph.D., FACSM WORLDWIDE SURVEY OF FITNESS TRENDS FOR 2019, ACSM's Health & Fitness Journal: 11/12 2018 -Volume 22 - Issue 6 - p 10-17)

### Diagnostic:

The lack of objective and easy biomarkers in sleep medicine is one of the key reasons for a lengthy and costly diagnostic process for sleep patients. Sometimes, this can also result in wrong or sub-optimal treatment decisions. Our technology will finally enable sleep doctors to diagnose 2-5 million patients in Germany with sleep-wake disorders quicker and more accurately (e.g. jet lag disorder or shift work disorder). In addition, the BODYTIME method will serve as a practical and fast tool for differential diagnosis of the 4-5 million chronic insomnia and hypersomnia patients in Germany (AWMF register Nr. 063/001, S3 Guideline Non-restorative sleep/sleep disorder).

### Treatment (chronotherapy)

Physicians diagnose and administer medicine as needed, although the best time to take a particular drug is rarely considered. Yet, increasing evidence shows that our body clock orchestrates 24-hour circadian rhythms in vital cardio-metabolic, endocrine, immunologic, and behavioral functions. Time therefore adds an important dimension to medicine. Chronotherapy aims to incorporate knowledge of the 24-hour biological rhythms to enhance diagnosis and treatment for different diseases. Several studies (e.g. in patients with colorectal cancer) have shown that time-dependent drug administration can in fact improve the drug's efficacy and reduce its toxicity and side effects, e.g. colorectal cancer (Lynne Peeples, Nature 556, 290-292 (2018)). Until now, the greatest barrier for chronotherapy has been the accurate, practical, and quick determination of an individual's body clock (chronotype). While the field of chronotherapy is still somewhat young, we consider our technology will help improve the selection of times of when medicine is taken, thereby enhancing therapeutic effect.

## Claims

1. A method for determining a circadian rhythm of a human subject, comprising
a. Providing a single sample comprising RNA from said subject, and
b. Quantifying an RNA expression level of multiple genes in said sample, said multiple genes comprising at least one gene selected from at least two of the following circadian phase gene groups: an early-day gene group, a day gene group, an evening gene group, and a night gene group, and at least one gene selected from a group of amplitude prediction genes, and
c. Determining the circadian rhythm based on the expression levels quantified in step b from said single sample.

2. The method according to claim 1, wherein said sample is a hair root, oral mucosa, or saliva sample, preferably a hair root sample.

3. The method according to any of the preceding claims, wherein said method comprises determining an amplitude and a phase of the circadian rhythm of the subject.

4. The method according to any of the preceding claims, wherein the method comprises normalizing the determined expression level of each circadian phase gene of claim 1 to an expression level of at least one housekeeping gene or to a mean expression level of all determined gene expression levels.

5. The method according to any of the preceding claims, comprising comparing the quantified (optionally normalized) RNA expression level of each gene to a predetermined gene expression level (such as maximum or minimum) for each gene and a corresponding phase in said human subject.

6. The method according to any of the preceding claims, comprising determining an amplitude of the circadian rhythm of the subject, wherein the expression level of the one or more genes selected from the group of amplitude prediction genes correlates with an amplitude of the circadian rhythm of the subject.

7. The method according to the preceding claim, wherein the amplitude provides an assessment and/or grading of the lifestyle of the human subject in relation to the determined circadian rhythm.

8. The method according to any of the preceding claims, wherein the circadian rhythm of the human subject is determined as one or more circadian rhythm types with a defined circadian phase of optimal and/or recommended physical activity of the subject.

9. The method according to any of the preceding claims, wherein the determined circadian rhythm, preferably the determined phase of the circadian rhythm, provides recommendations for physical activity of the subject, comprising one or more of recommended going to bed times, sleeping times, working times, mealtimes, coffee or tea drinking times, sports times, intellectual task times, creative task times, light exposure times, medical intervention times and/or medical diagnosis times.

10. The method according to any of the preceding claims, wherein said method comprises calculating the amplitude and the phase of the circadian rhythm of the subject using a biomathematical model, preferably Zeitzeiger, Lasso and/or partial least square, comprising:
a) Inputting the gene expression levels of the human subject quantified in step b. of claim 1,
b) Loading at least one training data set, wherein said training data set comprises data entries of reference gene expression levels (from other subjects), comprising gene expression levels from circadian phase genes and amplitude prediction genes, wherein each data entry comprises a gene label, a weight factor, and an amplitude score and/or time parameters,
c) Determining a circadian phase value for the subject by applying the weight factor of each circadian phase gene from the data entries in step b) to each circadian phase gene expression level inputted in step a),
d) Determining an amplitude value for the subject by applying the weight factor of amplitude prediction genes from the data entries in step b) to each amplitude prediction gene expression level inputted in step a),
e) Computing the phase of the circadian rhythm by applying a time point of sample collection for the sample according to claim 1 to the phase value from step c) and matching the phase value to the time parameters of the training data set from step b), and
f) Computing the amplitude of the circadian rhythm by matching the amplitude value from step c) with the amplitude score of the training data set from step b).

11. The method according to any of the preceding claims,
wherein a gene from the circadian phase early gene group is selected from an early morning gene group and/or late morning gene group, and comprises one or more genes of:
- An early morning gene selected from TEF, AC016727.1, TLR5, PDK1, HLF, PER3, PER2, ZBTB42, PER1, POLR2J4, SLC6A6, DUBR, BORCS6, SPRR2B and/or CRY2, and/or
- A late morning gene selected from PIF1, TRIM35, LRAT, HERC3, ALDH3A1 and/or RPL23AP7,
and/or
wherein a gene from the circadian phase day gene group is selected from an early afternoon gene group and/or late afternoon gene group, and comprises one or more genes of:
- An early afternoon gene selected from TGM5, TNS1, RORC, KLRG2, CRY1, and/or B3GNT2, and/or
- A late afternoon gene selected from MINOS1, ANO7L1, LRRC37A3, PSMG3-AS1, AC011476.3, SPRR2A, KRT15, CAMKMT, GNB1L, SERPINE2, SAP30, and/or PLEKHF1.
and/or
wherein a gene from the circadian phase evening gene group is selected from an early evening gene group and/or late evening gene group, and comprises one or more genes of:
- An early evening gene selected from PGF, ARNTL, ARSJ, NPAS2, SPRY2 and/or URB1-AS1, and/or
- A late evening gene selected from ZNF510, CLEC11A, GLI2, NDUFAF4, RBM18, ZNF669, DlO3OS, PIBF1, CDPF1, P4HA1, PRR34-AS1 and/or ZNF749.
and/or
wherein a gene from the circadian phase night gene group is selected from an early night gene group and/or a late night gene group, and comprises one or more genes of:
- An early night gene selected from RRAD, POLR3GL, SLC22A15, NR1H3, AC048341.2, STXBP4, CD34, MAP3K14, NME7, SCGB2A2, LAYN, C1orf123, RAI14, FBLN7, PDE6B, GOT1, PDSS1, POLR2I and/or C12orf66, and/or,
- Late night gene selected from NR1D1, CDC25B, ZSCAN31, VWF, TDRKH, DBP, AL391121.1, MOSPD2, CNN1, CDCA3, NR1D2, CIART, ZNF296 and/or U2AF1.

12. The method according to any of the preceding claims, wherein the amplitude prediction gene is selected from one or more of DUBR, GL12, MOSPD2, SPRY2, TLR5, ZNF296, ZNF749 and/or HERC3.

13. The method according to any of the preceding claims, wherein the determined amplitude and/or circadian phase indicate means to prevent and/or treat a circadian rhythm related disorder, comprising one or more of a disturbance in health or mental conditions, for example an advanced or delayed sleep-wake phase disorder, a non-24-hour sleep-wake rhythm disorder, a shift work disorder, and/or a jet lag disorder.

14. The method according to any of the preceding claims, wherein said RNA expression is determined using a method selected from the group consisting of NanoString, quantitative PCR, microarray, or sequencing, preferably NanoString.

15. A method for providing optimal and/or recommended physical activity times for a subject comprising carrying out the method according to any of the preceding claims, wherein said physical activity times comprise one or more of recommended sleeping times, working times, mealtimes, sport times, intellectual task times, light exposure times, medical intervention times and/or medical diagnosis times.

16. A computer readable storage medium comprising instructions to configure a processor to perform a method according to any of the preceding claims,
the method preferably comprising:
- a biomathematical model with quantitative RNA expression values as input values determined in a method according to any one of the preceding claims, and
- a training data set comprising data entries of reference gene expression levels (from other subjects), comprising gene expression levels from circadian phase genes and amplitude prediction genes, wherein each data entry comprises a gene label, a weight factor, and an amplitude score and/or time parameters.
